(19) Europäisches Patentamt
European Patent Office
Office européen des brevets

(11) EP 0 737 668 B1

(12) **EUROPÄISCHE PATENTSCHRIFT**

(45) Veröffentlichungstag und Bekanntmachung des Hinweises auf die Patenterteilung:
**10.02.1999 Patentblatt 1999/06**

(51) Int Cl.[6]: **C07C 209/86**, C07C 209/84, C07C 211/50

(21) Anmeldenummer: **96104872.5**

(22) Anmeldetag: **27.03.1996**

(54) **Fraktionierung und Reinigung von aromatischen Polyamingemischen und deren Verwendung**

Fractionation and purification of mixtures of aromatic polyamines and their use

Fractionnement et purification de mélanges de polyamines aromatiques et leur utilisation

(84) Benannte Vertragsstaaten:
**BE DE ES FR GB IT NL PT**

(30) Priorität: **07.04.1995 DE 19513163**

(43) Veröffentlichungstag der Anmeldung:
**16.10.1996 Patentblatt 1996/42**

(73) Patentinhaber: **BAYER AG**
**51368 Leverkusen (DE)**

(72) Erfinder:
- **Knöfel, Hartmut, Dr.**
**51519 Odenthal (DE)**
- **Brockelt, Michael**
**51379 Leverkusen (DE)**

(56) Entgegenhaltungen:
**EP-A- 0 288 892**
**DE-A- 1 568 087**
**DE-A- 2 528 694**

- **BECKER H. ET AL.: "Organikum" 1976 , VEB DEUTSCHER VERLAG DER WISSENSCHAFTEN , BERLIN XP002005624 * Seite 74 - Seite 82 ***

EP 0 737 668 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft ein Verfahren zur Fraktionierung und Reinigung von aromatischen Polyamingemischen und deren Verwendung.

Die Herstellung von aromatischen Polyaminen und Polyamingemischen, insbesondere der Diphenylmethanreihe, wird in zahlreichen Patentmeldungen und Patenten beschrieben, ebenso die Verwendung dieser Produkte. Herausragende Bedeutung kommt dabei der Verwendung dieser Produkte als Rohstoffe für die Herstellung von Isocyanaten zu, in der Regel durch Umsetzung der Polyamingemische mit Phosgen nach den allgemein üblichen und bekannten Methoden.

Die dabei resultierenden Isocyante bzw. Isocyanatgemische fallen in vielen Fällen aber nicht in der Form und Zusammensetzung an, wie sie auf der Isocyanatstufe bevorzugt weiterverwendet werden, sondern müssen zuvor durch teilweise aufwendige Aufarbeitungs- und Trennverfahren in die venvendungsgerechte Form übergeführt werden. Geeignete Polyaminvorstufen, die weniger aufwendig in die Isocyanatverwendungsformen gebracht werden können, sind in vielen Fällen verfahrenstechnisch schwierig oder gar nicht zugänglich oder wirtschaftlich unattraktiv herzustellen.

Beispielhaft ist die Gewinnung des für die Herstellung hochwertiger Polyurethanwerkstoffe wichtigen 4,4'-Diisocyanato-diphenylmethans, dessen Aminvorstufe in der Regel aus Anilin und Formaldehyd nur gemeinsam mit Isomeren, insbesondere dem 2,4'-Isomeren, und höherfunktionellen Polyaminen gewonnen werden kann. Diese Bestandteile sind zwar die Grundlage für ebenfalls begehrte Isocyanate, doch ist die Auftrennung der Rohisocyanate in die für die Weiterverwendung geeigneten Isocyanate bzw. Isocyanatgemische nicht einfach.

In der Regel werden zunächst ein Teil der Zweikernverbindungen von dem Rest abgetrennt. Anschließend wird aus der Zweikernfraktion in einem viele Trennstufen erfordernden zweiten Destillationsschritt das 4,4'-Diisocyanato-diphenylmethan von den anderen Isomeren befreit.

Das 2,4'-Isomere in angereicherter Form hat selbst in neuerer Zeit zunehmende Bedeutung als Polyurethanrohstoff erlangt, und kann nur mit beträchtlichem destillativen Aufwand gegenüber dem 4,4'-Isomeren angereichert und von dem gegebenenfalls vorhandenen 2,2'-Isomeren befreit werden.

Isomerentrennverfahren oder Anreicherungsverfahren innerhalb der Fraktion der höherkernigen Homologen bzw. der höherfunktionellen Bestandteile der Amine wie auch der Isocyanate der Diphenylmethanreihe sind praktisch nicht bekannt.

Zunehmendes Interesse findet auch das 4,4'-Diamino-diphenylmethan als Rohstoff für das Di-(4-isocyanatocyclohexyl)-methan, die kernhydrierte Form des 4,4'-Diisocyanato-diphenylmethans, wobei die Bereitstellung geeigneter aromatischer Polyamingemische für die Hydrierstufe mit einem möglichst hohen Gehalt an 4,4'-Diamino-diphenylmethan bei gleichzeitig einem möglichst geringen Anteil an 2,4'-Diamino-diphenylmethan sehr aufwendig ist.

Es ist bekannt, daß Amine durch partielle Überführung in ihre Salze in bestimmten Fällen getrennt werden können, wobei u.a. die unterschiedlichen Basenstärken genutzt werden. Dabei handelt es sich in der Regel um Monoamine mit stark unterschiedlichen Basenstärken.

Auch für aromatische Polyamingemische, insbesondere der Diphenylmethanreihe, sind solche Disproportionierungseffekte in zweiphasigen Systemen bereits beschrieben (DE-A 2238319 und DE-A 2528694).

Die Effekte sind infolge der in einem solchen Gemisch vorhanden zahlreiche Komponenten, deren Aminogruppen sich vom Typ her - praktisch alle sind Arylaminogruppen - kaum unterscheiden, nicht besonders groß und ausgeprägt, um für eine direkte Nutzung mit einfachen Mitteln interessant zu sein.

Aufgabe war es, ein Verfahren zur Verfügung zu stellen, das es gestattet, aromatische Polyamingemische in einfacher Weise zu fraktionieren bzw. zu reinigen, so daß Isomere in reiner Form oder in angereicherter Form anfallen.

Diese Aufgabe konnte durch das erfindungsgemäße Verfahren, welches aufgrund der erfindungsgemäßen Ausführung überraschend hohe Trennleistung bei der Fraktionierung von aromatischen Polyamingemischen, insbesondere der Diphenylmethanreihe, erzielt, und dabei in der Wirkung weit über die bekannten Effekte des Standes der Technik hinausgeht, gelöst werden.

Bei der erfindungsgemäßen Fraktionierung von aromatischen Polyamingemischen werden andere, unterschiedlich zusammengesetzte Polyamingemische gewonnen.

Bei diesen abgeieiteten Polyamingemischen kann es sich um solche handeln, die auf bekannten Synthesewegen nur sehr aufwendig zugänglich sind. Dabei kann es sich auch um Polyamingemische handeln, die für eine vereinfachte Herstellung der Isocyanate besser geeignet sind, als die bekannten und technisch gut herzustellenden Polyamingemische, indem sie z. B. auf der Isocyanatstufe schwierig durchzuführende Isomerentrennungen durch entsprechende Isomerenanreicherungen auf der Aminstufe vorwegnehmen. Solche Gemische können auch völlig neuartige, weil nach dem Stand der Technik nicht darstellbare Polyamingemische sein, die zu völlig neuartigen Isocyanaten führen.

Andererseits kann das erfindungsgemäße Verfahren dazu genutzt werden, aus beliebigen, d.h. auch aus wiedergewonnenen Polyamingemischen, die sich durch Verunreinigung oder durch nichtstatistische, d. h. selektive Verluste bei einzelnen Komponenten bei der Wiedergewinnung von den ursprünglich eingesetzten Polyaminen oder

Isocyanaten unterscheiden, Produktfraktionen zu gewinnen, welche dem Standard oder den Ausgangspolyaminen entsprechen.

Schließlich kann das erfindungsgemäße Verfahren dazu genutzt werden, synthesebedingte und im Endprodukt unerwünschte Neben- und Zwischenprodukte, mitzufraktionieren und in einer Produktfraktion ab - und in einer anderen entsprechend anzureichern, gegebenenfalls in einer eigenen Fraktion auszuschleusen.

Es handelt sich bei der vorliegenden Erfindung um ein breit anwendbares Verfahren, mit welchem die Aufgabe der Fraktionierung und Reinigung von aromatischen Di- und Polyamingemischen, insbesondere der Diphenylmethanreihe gelöst werden kann.

Gegenstand der Erfindung ist ein Verfahren zur Fraktionierung und Reinigung von aromatischen Polyamingemischen, insbesondere von Polyamingemischen der Diphenylmethanreihe, welches dadurch gekennzeichnet ist, daß man

a) das Polyaminausgangsgemisch (A) in einem zweiphasigen System, bestehend aus (i) einer hydrophoben Lösungsmittel phase (B), die im wesentlichen aus hydrophobem Lösungsmittel und gegebenenfalls einem aromatischen Hilfsamin, welches in Wasser praktisch unlöslich ist und unter Normaldruck einem mindestens 20°C unter dem Siedepunkt der am niedrigsten siedenden Komponente des Ausgangsgemisches und mindestens 20°C oberhalb des Siedepunktes des Lösungsmittels liegenden Siedepunkt aufweist, und gegebenenfalls aus Polyamin besteht, und (ii) einer wäßrigen Phase (C), bestehend im wesentlichen aus Wasser, einer starken Säure und zumindest teilweise in der Salzform vorliegendem Hilfsamin, sowie gegebenenfalls zumindest teilweise in der Salzform vorliegenden Polyaminen, unter Zuhilfenahme einer nach dem Gegenstromprinzip arbeitenden Extraktionsstufe (4) unter Durchmischung der Phasen verteilt, indem man das Ausgangspolyamingemisch vorzugsweise über die wäßrige Phase in die Extraktionsstufe (4) einbringt und die die Extraktionsstufe (4) verlassende organische Phase (D)

b) gegebenenfalls zumindest teilweise über eine zwischengeschaltete Extraktionsstufe (3) und/oder

c) gegebenenfalls unter Abtrennung eines Teilstromes vor oder nach der gegebenenfalls durchlaufenden Extraktionsstufe (3) und Rückführung des abgetrennten Teilstromes zur Extraktionsstufe (4) über eine vorgeschaltete Extraktionsstufe (2)

d) in einer mehrstufigen Destillation (7.1), (7.2) in eine erste, wiederverwendete Fraktion (E), bestehend im wesentlichen aus hydrophobem Lösungsmittel und gegebenenfalls Hilfsamin, eine zweite Fraktion (F), bestehend im wesentlichen aus Hilfsamin und gegebenenfalls hydrophobem Lösungsmittel und einem Destillationsrückstand (G), bestehend im wesentlichen aus einer ersten Polyaminfraktion, auftrennt und

e) die die Extraktionsstufe (4) verlassende wäßrige Phase (H) in eine Extraktionsstufe (5) leitet, in welcher nach dem Prinzip der Gegenstromextraktion eine Extraktion der wäßrigen Phase mit einer aus hydrophobem Lösungsmittel und Hilfsamin bestehenden Lösungsmittelphase (J) erfolgt, wobei die an Polyamin verarmte wäßrige Phase (K) resultiert, und

f) die in der Extraktionsstufe (5) anfallende organische Phase (L) zumindest teilweise, gegebenenfalls nach Durchlaufen einer Waschstufe (8.0), in einer mehrstufigen Destillation (8.1), (8.2) in eine erste Fraktion (M), bestehend im wesentlichen aus hydrophobem Lösungsmittel und gegebenenfalls Anteilen an Hilfsamin, und eine zweite Fraktion (N) bestehend im wesentlichen aus Hilfsamin und gegebenenfalls Anteilen an hydrophobem Lösungsmittel und einen Destillationsrückstand (O), bestehend im wesentlichen aus einer zweiten Polyaminfraktion, auftrennt und

g) die in der Extraktionsstufe (5) anfallende wäßrige Phase (K) zumindest teilweise,

h) gegebenenfalls zumindest teilweise über eine zwischengeschaltete Destillationsstufe (9)

i) in eine Extraktionsstufe (6) leitet und im Gegenstrom mit einer organischen Phase (P) extrahiert, bestehend im wesentlichen aus hydrophobem Lösungsmittel und gegebenenfalls höchstens soviel Hilfsamin, daß in (6) eine gegenüber der eingesetzten wäßrigen Phase (K) an Arylamin verarmte wäßrige Phase (Q) resultiert, die man

k) gegebenenfalls zumindest teilweise über eine zwischengeschaltete Destillationsstufe (9)

l) mit dem gegebenenfalls vorhandenen Resten von (Q) und (K) vereinigt und als Mengenstrom (C) wiederverwendet, indem man Mengenstrom (C)

m) gegebenenfalls zumindest teilweise über eine zwischengeschaltete Extraktionsstufe (3) und/oder

n) gegebenenfalls zumindest teilweise zunächst über eine vorgeschaltete Extraktionsstufe (2) und anschließend über eine gegebenenfalls vorhandene Extraktionsstufe (3)

o) zur Extraktionsstufe (4) zurückgeführt und dort gegebenenfalls nach Zugabe von Wasser und/oder Hilfsamin als Mengenstrom (C) wiederverwendet und man

p) die in der Extraktionsstufe (6) anfallende organische Phase (R), gegebenenfalls nach Zugabe von weiterer, im wesentlichen aus Hilfsamin bestehender organischer Phase aus Mengenstrom (S) als Extraktionsmittelphase (J) der Extraktionsstufe (5) zuführt.

Kennzeichnend für die Ausführung des erfindungsgemäßen Verfahrens ist, daß
der wiederverwendete Mengenstrom (C) einen höheren Protonierungsgrad aufweist als die die Extraktionsstufe (4) verlassende wäßrige Phase (K), gegebenenfalls bis zu 100 %.

Bevorzugt wird das Verfahren so durchgeführt, daß man

b) die in der Extraktionsstufe (4) anfallende organische Phase (D) zumindest teilweise in einer zwischengeschalteten Extraktionsstufe (3) mit zumindest einer Teilmenge des Mengenstrom (C) im Gegenstrom extrahiert und/oder mit zumindest einer Teilmenge der in der gegebenenfalls vorhandenen vorgeschalteten Extraktionsstufe (2) anfallenden wäßrigen Phase (Z) im Gegenstrom extrahiert,
die in der zwischengeschalteten Extraktionsstufe (3) resultierende wäßrige Phase (U) der Extraktionsstufe (4) zuführt und
die in der zwischengeschalteten Extraktionsstufe (3) anfallende organische Phase (V) der Aufarbeitungsstufe (7) zuführt.

Das erfindungsgemäße Verfahren wird besonders bevorzugt so durchgeführt, daß man

c) einen Teilstrom der die Extraktionsstufe (4) verlassenden organischen Phase (D) und/oder
einen Teilstrom der die gegebenenfalls vorhandene zwischengeschaltete Extraktionsstufe (3) verlassenden organischen Phase abtrennt
und in einer vorgeschalteten Extraktionsstufe (2) mit einer Teilmenge, vorzugsweise mit der Gesamtmenge der als Mengenstrom (C) zur Verfügung stehenden wäßrigen Phase im Gegenstrom extrahiert,
den in der Extraktionsstufe (2) eingesetzten organischen Mengenstrom (W) so bemißt, daß in (2) ein möglichst weitgehender Übergang des im besagtem organischen Mengenstrom enthaltenen Polyamins in die wäßrige Phase erfolgt,
die in der vorgeschalteten Extraktionsstufe (2) resultierende wäßrige Phase (Z) gegebenenfalls nach Zugabe von Wasser aus Mengenstrom (Y) und/oder Hilfsamin zumindest teilweise der Extraktionsstufe (3) zuführt und die in der vorgeschalteten Extraktionsstufe (2) anfallende an Polyamin verarmte organische Phase der Extraktionsstufe (4) zuführt.

Eine weiter verbesserte und daher bevorzugte Ausführungsform des erfindungsgemäßen Verfahrens wird dadurch erreicht, daß man

k) die die Extraktionsstufe (5) verlassende wäßrige Phase (K) vor ihrer Wiedervenvendung zumindest teilweise und/oder die die Extraktionsstufe (6) verlassende wäßrige Phase (Q) vor ihrer Wiederverwendung zumindest teilweise destillativ (9) von einem Teil (X) des in ihr enthaltenen Wassers befreit, dieses gegebenenfalls zum Waschen (7.0) des der destillativen Aufarbeitung (7.1), (7.2) zugeführten Teiles der die Extraktionsstufe (4) verlassenden organischen Phase (D) und/oder der die Extraktionsstufe (3) verlassenden organischen Phase (V) und/oder zum Waschen (8.0) des der destillativen Aufarbeitung (8.1), (8.2) zugeführten Teiles der die Extraktionsstufe (5) verlassenden organischen Phase (L) zwecks Entfernens von Säurespuren verwendet, das hierbei anfallende Wasser (Y) an geeigneter Stelle in die wäßrige Phase zurückführt, die resultierende konzentrierte wäßrige Phase mit dem gegebenenfalls verbliebenen Resten von (K) und (Q) vereinigt und als Mengenstrom (C) der Wiederverwendung zuführt.

Als Hilfsamin wird vorzugsweise Anilin eingesetzt und als Polyamingemisch der Diphenylmethanreihe bevorzugt ein Polyamingemisch, wie es bei der säurekatalysierten Anilin/Formaldehyd-Kondensation anfällt.

Die derart behandelten Polyamingemische, also die mit dem erfindungsgemäßen Verfahren erzeugten Fraktionen werden zur Herstellung der entsprechenden aromatischen Polyisocyanatgemische und zur Herstellung von Polyurethankunststoffen verwendet.

Außerdem können die nach dem erfindungsgemäßen Verfahren erzeugten Fraktionen zur Herstellung der entsprechenden kernhydrierten Polyamine oder als Vernetzer und als Epoxidhärter verwendet werden.

Die aus den fraktionierten Polyamingemischen hergestellten entsprechenden Polyisocyanate werden bevorzugt zur Herstellung von PU-Schaumstoffen eingesetzt.

Ausgangsgemische sind beispielsweise technische Arylamingemische, wie sie bei der Herstellung aus den Ausgangsverbindungen oder wie sie bei der Wiedergewinnung anfallen.

Beispiele von Ausgangsarylamingemischen, für deren Fraktionierung und Reinigung das erfindungsgemäße Verfahren besonders geeignet ist, sind

1. Polyamingemische der Diphenylmethanreihe, wie sie bei der Kondensation und säurekatalysierten Umlagerung von Anilin mit Formaldehyd entstehen,

2. Polyamingemische der Diphenylmethanreihe, wie sie bei der säurekatalysierten Kondensation von substituierten Anilinen mit Formaldehyd anfallen,

3. Polyamingemische der Diphenylmethanreihe, wie sie bei der Mischkondensation von substituierten Anilinen untereinander und/oder mit Anilin mit Formaldehyd anfallen,

4. Polyamingemische der Diphenylmethanreihe, wie sie bei der Kondensation, auch der Mischkondensation von substituierten Anilinen und/oder Anilin mit Aldehyden und/oder Ketonen anfallen,

5. Polyamingemische der Diphenylmethanreihe, wie sie bei der Nitrierung und anschließenden Reduktion von Di- und/oder Polyarylmethanen entstehen; unter Polyarylmethanen werden hier insbesondere die Benzylhomologen des Diphenylmethans verstanden,

6. Polyamingemische der Diphenylmethanreihe, wie sie bei der Kondensation von Monoarylmonoaminen (z. B. Anilin, substituierte Aniline) und/oder Monoaryldiaminen (Phenylendiamine, substituierte Phenylendiamine) mit Aldehyden, Ketonen insbesondere Formaldehyd, und säurekatalysierter Umlagerung entstehen und

7. Polyamingemische der Triphenylmethanreihe, wie sie z.B. bei der Nitrierung und anschließenden Reduktion von Triphenylmethan, insbesondere alkylsubstituierten Triphenylmethanen und seinen höherkernigen, insbesondere Benzylhomologen entstehen.

Bei den zum Einsatz gelangenden hydrophoben Lösungsmitteln handelt es sich um inerte Lösungsmittel des Siedepunktbereiches von 30 - 280°C, vorzugsweise von 80 - 200°C, wie beispielsweise Chlorbenzol, Dichlorbenzol, Benzol, Toluol, Ethylbenzol, Cumol, Xylol, Dichlorethan, Chloroform und Tetrachlorkohlenstoff. Vorzugsweise werden Xylole, d. h. technische Xylolgemische, insbesondere o-Xylol, Toluol, Ethylbenzol, Cumol und Chlorbenzol eingesetzt.

Bevorzugt werden solche Lösungsmittel verwendet, die ein gutes Lösungsvermögen für die eingesetzten Polyamingemische aufweisen.

Bei den eingesetzten Säuren handelt es sich um wasserlösliche Protonsäuren mit einem unter 2,5,vorzugsweise unter 1,5 liegenden pKA-Wert. Beispiele hierfür sind Salzsäure, Bromwasserstoffsäure, Schwefelsäure, Trifluoressigsäure, Methansulfonsäure oder Phosphorsäure.

Bevorzugt eingesetzt werden Salzsäure und Methansulfonsäure. Die genannten Säuren können auch im Gemisch mit sauren oder neutralen Salzen derartiger Säuren, wie z.B. den entsprechenden Ammoniumsalzen oder auch den entsprechenden Alkali salzen, eingesetzt werden. Die genannten Säuren werden nicht in freier Form eingesetzt, sondern liegen in dem erfindungsgemäßen Kreislaufsystem in Form der entsprechenden Ammoniumsalze der in den wäßrigen Kreislaufsystem befindlichen Basen vor. Das sind in der Regel Polyamingemische von der Art der Ausgangsgemische und/oder die verwendeten Hilfsamine.

Als Hilfsamin finden in der Regel Monoarylamine, wie z. B. Anilin und/oder am Kern und/oder am Stickstoff alkylsubstituierte Anilinderivate, Verwendung. Bevorzugt werden primäre Aniline eingesetzt, besonders bevorzugt ist Anilin.

Das erfindungsgemäße Verfahren kann sowohl diskontinuierlich als auch kontinuierlich durchgeführt werden. Bevorzugte Ausführungsform ist die kontinuierliche Arbeitsweise. Dabei wird das Verfahren in allen Stufen unter dem Eigendruck des Systems und vorzugsweise in einer Inertgasatmosphäre (Stickstoff) durchgeführt.

Das erfindungsgemäße Verfahren kann zur Steigerung des Anreicherungs- bzw. korrespondierenden Abreicherungseffektes mit jeder der anfallenden Produktfaktionen wiederholt werden.
Das erfindungsgemäße Verfahren kann sowohl mit drei (Abb. 1) als auch mit vier (Abb. 2 und Abb. 3) oder mit fünf Extraktionsstufen (Abb. 4) durchgeführt werden.

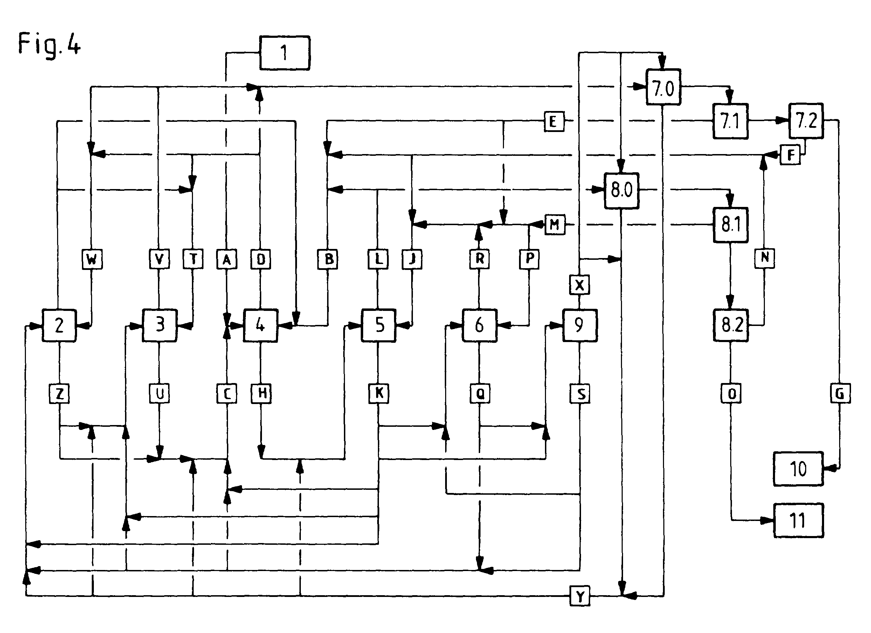

Die in Abb. 1 - 4 dargestellten Fließdiagramme dienen der weiteren Erläuterung des erfindungsgemäßen Verfahrens. In diesen Abbildungen bedeuten:

(1) einen Tank für das Ausgangsarylamingemisch

(2) eine (vorgeschaltete) Extraktionsstufe

(3) eine (zwischengeschaltete) Extraktionsstufe

(4) eine (erste) Extraktionsstufe

(5) eine (vorletzte) Extraktionsstufe

(6) eine (letzte) Extraktionsstufe

(7) eine Aufarbeitungsstufe bestehend aus

(7.0) einer Waschstufe,

(7.1) einer ersten Destillationsstufe einer Mehrstufendestillation,

(7.2) einer letzten Destillationsstufe einer Mehrstufendestillation,

(8) eine weitere Aufarbeitungsstufe bestehend aus

(8.0) einer Waschstufe,

(8.1) einer ersten Destillationsstufe einer weiteren Mehrstufendestillation,

(8.2) einer letzten Destillationsstufe einer weiteren Mehrstufendestillation,

(9) einen Wasserverdampfer

(10) einen Tank für ein Verfahrensprodukt

(11) einen Tank für ein weiteres Verfahrensprodukt

Die Bezugszeichen A - Z bezeichnen die Mengenströme auf die nachstehend und in den Beispielen Bezug genommen wird.
Die vorgeschaltete Extraktionsstufe (2) wird im allgemeinen als mehrstufig wirkender Extraktor ausgeführt.
Die gegebenenfalls zwischengeschaltete Extraktionsstufe (3) besteht im einfachsten Falle ebenfalls aus einer Mischer-Scheidereinheit, vorzugsweise kommen jedoch auch hier mehrstufig wirkende Extraktionseinheiten zum Einsatz.
Bei der Extraktionsstufe (4) handelt es sich im einfachsten Falle um eine einstufig wirkende Mischer-Scheidereinheit, vorzugweise kommen jedoch mehrstufig wirkende Extraktionseinheiten zum Einsatz.
Die Extraktionsstufen (5) und (6) werden im allgemeinen als mehrstufig wirkende Extraktoren ausgeführt.
Die Aufarbeitungsstufen (7) und (8) dienen der Abtrennung der Polyaminfraktionen, welche als Destillationsrückstände anfallen und als Verfahrensprodukte (G) und (O) in den Tanks (10) und (11) isoliert werden, und der Wiedergewinnung des eingesetzten hydrophoben Lösungsmittels und des verwendeten Hilfsamins als Destillate.
Im allgemeinen ist die Wiedergewinnung von hydrophobem Lösungsmittel und Hilfsamin mit einer weitgehenden, gegebenenfalls vollständigen Auftrennung beider Komponenten verbunden und erfolgt unter Gewinnung von zumindest an Hilfsamin weitgehend verarmten, gegebenenfalls von Hilfsamin weitgehend befreiten hydrophobem Lösungsmittel in Form der Destillatströme (E) und (M) einerseits und von zumindest an hydrophobem Lösungsmittel weitgehend verarmten, gegebenenfalls von hydrophobem Lösungsmittel weitgehend befreiten Hilfsamin in Form der

Destillatströme (F) und (N), in der Regel zusammengefaßt im Mengenstrom (S).

Es hat sich als zweckmäßig erwiesen, die den Destillationsstufen zugeführten organischen Phasen (D) bzw. (D') und (L) bzw. (L') vor ihrer destillativen Behandlung in vorgelagerten Waschstufen (7.0) und (8.0) durch Extraktion mit Wasser von anhaftenden Säurespuren zu befreien.

Die eigentliche Aufarbeitungsstufe (7) besteht im allgemeinen aus einer wenigsten zweistufigen Mehrstufendestillation, deren erste Stufe (7.1) ein gegenüber dem Zulaufprodukt (D) bzw. (D') von Polyarylamin befreites und an Hilfsamin verarmtes, gegebenenfalls von Hilfsamin befreites hydrophobes Lösungsmittel als Destillat (E) liefert und deren letzte Stufe (7.2) ein gegenüber (D) bzw. (D') von Polyarlyamin befreites und an hydrophobem Lösungsmittel verarmtes, gegebenenfalls von hydrophobem Lösungsmittel befreites Hilfsamin als Destillat (F) liefert.

Zusätzlich fällt in der letzten Destillatiosstufe (7.2) die im Mengenstrom (D) bzw. (D') enthaltene erste Polyaminfraktion des Ausgangsgemisches (A) als Destillationssumpf (G) an.

Auch die Aufarbeitungsstufe (8) besteht im allgemeinen aus einer wenigstens zweistufigen Mehrstufendestillation, deren erste Stufe (8.1) ein gegenüber dem Zulaufprodukt (L) bzw. (L') von Polyamin befreites und an Hilfsamin verarmtes, gegebenenfalls von Hilfsamin befreites hydrophobes Lösungsmittel als Destillat (M) liefert und deren letzte Stufe (8.2) ein gegenüber (L) bzw. (L') von Polyarylamin befreites und an hydrophobem Lösungsmittel verarmtes, gegebenenfalls von an hydrophobem Lösungsmittel befreites Hilfsamin als Destillat (N) liefert.

Die weitgehend, gegebenenfalls vollständige destillative Auftrennung von hydrophobem Lösungsmittel und Hilfsamin ist bei der Durchführung des erfindungsgemäßen Verfahrens bevorzugt, das gilt insbesondere für die Destillatfraktionen (M) und (E).

Bei der Destillationsstufe (9) handelt es sich um eine Vorrichtung, mit welcher der wäßrigen Phase des Systems oder einem Teilstrom der wäßrigen Phase destillativ Wasser entzogen werden kann.

Ein solcher Schritt ist für die Durchführung des erfindungsgemäßen Verfahrens grundsätzlich nicht erforderlich, wegen der sich ergebenden Vorteile sind die Ausführungsformen unter Einschluß einer Wasserdestillationsstufe (9) jedoch bevorzugt.

Bei der wäßrigen, die Säure enthaltenden Phase liegt praktisch ein geschlossenes Kreislaufsystem vor, so daß die Stufe (9) grundsätzlich an einer beliebigen Stelle dieses Kreislaufsystems eingeschoben werden kann. Die Position von Stufe (9) in Anschluß an die Extraktionsstufe (6) und vor Eintritt in die Extraktionsstufe (2), (3) oder (4) ist die vorteilhafteste und daher die bevorzugte Ausführungsform.

Die entnommene Wassermenge (X) wird gegebenenfalls nach ihrer Aufteilung in Teilströme und deren unterschiedlicher Verwendung in Form des Mengenstromes (Y) insgesamt oder in Teilströmen dem System an geeigneter Stelle wieder zugeführt, so daß ein erweitertes und gegebenenfalls verzweigtes in sich geschlossenes wäßriges Kreislaufsystem entsteht.

Dieses schließt auch die Waschstufen (7.0) und/oder (8.0) mit ein. Letztere sind ein oder mehrstufig wirkende nach dem Gegenstromprinzip arbeitende Extraktionsstufen. In Waschstufe (7.0) wird die organische Phase (D) bzw. (D') mit einem Teilstrom von (X) von anhaftenden Säurespuren befreit, in der Waschstufe (8.0) geschieht das Analoge mit der organischen Phase (L) bzw. (L') unter Verwendung eines anderen Teilstromes von (X).

Das mit hydrophobem Lösungsmittel und Hilfsamin contaminierte Destillat (X) ist für die Waschstufen (7.0) und (8.0) bestens geeignet. Die resultierenden Waschwässer weisen in der Regel eine sehr viel geringere Säurekonzentration auf als der eigentliche Säurekreislauf, so daß diese problemlos in Form des Mengenstromes (Y) oder seiner Teilströme recyclisiert werden können, gegebenenfalls kann eine Destillatteilmenge von (X) an den Waschstufen vorbei nach (Y) geführt und zur Aussteuerung eines unterschiedlichen Wassergehaltes in den einzelnen Extraktionsstufen verwendet werden.

Die praktisch quantitative Kreislaufführung der verwendeten Säure ermöglicht den Einsatz kostspieliger Säuren wie z. B. Methansulfonsäure, die wiederum wegen ihrer geringeren Korrosionsneigung die Verwendung kostengünstiger Werkstoffe in den Apparaten des erfindungsgemäßen Verfahrens gestattet.

Es hat sich als zweckmäßig erwiesen, den Säuregehalt der wäßrigen Phase, unabhängig von dem sich in der wäßrigen Phase eines zweiphasigen Systems einstellenden unterschiedlichen Amingehalt, über eine sogenannte "Molarität" zu definieren.

Die Molarität wird festgelegt als theoretische Konzentration von zu 100 % protoniertem Amin (d. h. gleiche Anzahl von Säure- und Aminäquivalenten) in einem um den Anteil an nicht protoniertem Amin verminderten Volumen an wäßriger Phase gemäß der Formel

$$\text{"Molarität"} = \frac{\text{Mol 100 \% protoniertes Amin}}{\text{Vol. wäßrige Phase - Vol. unprotoniertes Amin}}$$

Die so definierte Molarität kann Werte bis 6 annehmen und wird je nach der der jeweiligen Ausführungsform zugrunde liegenden - hier produktbezogenen - Trennaufgabe in diesem Bereich gezielt variiert.

Auch innerhalb einer Ausführungsform des erfindungsgemäßen Verfahrens ist es gegebenenfalls vorteilhaft,

die einzelnen von der wäßrigen Phase durchlaufenen Verfahrensstufen, insbesondere die Extraktionsstufen (2) bis (6) mit unterschiedlicher Molarität in der wäßrigen Phase zu betreiben, indem der wäßrigen Phase zwischen den einzelnen Stufen Wasser entzogen oder zugeführt wird.

Nach oben wird dieser Arbeitsbereich praktisch begrenzt einerseits durch zunehmende Kristallisationsneigung der Aminsalze mit zunehmender Konzentration, insbesondere bei hohen Protonierungsgraden, und andererseits durch die zunehmende gegenseitige Löslichkeit der Phasen ineinander, insbesondere bei niedrigen Protonierungsgraden.

Gemäß einer Variante des erfindungsgemäßen Verfahrens erfolgt die Einspeisung des Ausgangspolyamingemisches (A) aus dem Vorratsbehälter (1) durch Vermischen mit dem Teilstrom (C) und Einführung des Gemisches in die Extraktionsstufe (4).

Im allgemeinen besteht der Mengenstrom (C) aus Wasser, einer starken Protonsäure, Hilfsamin und gegebenenfalls Polyamin.

Die Säure liegt in Form ihrer in Wasser gelösten Salze mit Hilfsamin und gegebenenfalls mit Polyamin vor. Die Summe der Aminogruppen von Hilfsamin und gegebenenfalls Polyamin liegen in (C) stets im stöchiometrischen Verhältnis oder im Überschuß vor, bezogen auf die Säure.

Der Protonierungsgrad beträgt in (C) im allgemeinen 40 bis 100 %, für das vorzugsweise als Hilfsamin verwendete Anilin liegt er vorzugsweise bei 60 bis 100 %.

Die für die jeweilige Ausführungsform des erfindungsgemäßen Verfahrens wohldefinierte und in engen Grenzen gemessene und geregelte Molarität des Mengenstromes (C) wird je nach der der jeweiligen Ausführungsform zugrunde liegenden - hier produktbezogenen - Trennaufgabe in einem weiten Bereich gezielt varriert.

Im allgemeinen hat die der Extraktionsstufe (4) des erfindungsgemäßen Verfahrens zugeführt wäßrige Phase (C) eine Molarität bis 6, vorzugsweise zwischen 0.5 und 4.0.

In der vorzugsweise mehrstufig betriebenen Extraktionsstufe (4) wird der mit (A) beaufschlagte Mengenstrom (C) und die organische Phase (B) unter inniger Durchmischung einander entgegengeführt.

Die organische Phase (B) besteht im allgemeinen neben hydrophobem Lösungsmittel aus Hilfsamin und/oder Polyamin, letzteres vorzugweise mit der Zusammensetzung des zweiten Verfahrensteilproduktes (O).

Bei der Verwendung einer organischen Phase (B) ohne Polyamin resultiert in der die Extraktionsstufe (4) verlassenden wäßrigen Phase (H) eine Polyaminfraktion, in welcher die relative Anreicherung der in dieser Phase bevorzugt enthaltenen Komponenten gezielt erhöht und maximiert werden kann, auf Kosten der Polyaminkonzentration in der wäßrigen Phase (H).

Polyamin als Bestandteil der organischen Phase (B) bewirkt, daß die die Verfahrensstufe (4) verlassenden Phasen (D) und (H) eine höhere und damit für die Durchführung des erfindungsgemäßen Verfahrens energetisch vorteilhaftere Polyaminkonzentration aufweisen, als bei der Verwendung einer organischen Phase (B) ohne Polyamin.

Durch die bevorzugte Verwendung eines Polyamins mit der Zusammensetzung des zweiten Teilproduktes (O) als Bestandteil der organischen Phase (B) kann auch auf einem höheren und damit vorteilhaften Konzentrationsniveau infolge Gleichgewichtseinstellung mit Selbstverstärkung des Trenneffektes die relative Anreicherung der in der die Trennstufe (4) verlassenden wäßrigen Phase (H) bevorzugt enthaltenen Polyaminkomponenten und damit der zweiten Polyaminfraktion (O) variiert und maximiert werden.

Im einfachsten Fall wird der Mengenstrom (B) gebildet aus dem von Polyamin (G) befreiten und an Hilfsamin weitgehend verarmten, als Destillat in Stufe (7.1) gewonnenen Mengenstrom (E) gegebenenfalls unter Zusatz einer Teilmenge von Mengenstrom (S), bestehend in wesentlichen aus an hydrophobem Lösungsmittel verarmten und von Polyamin befreitem Hilfsamin.

Vorteilhaft und bevorzugt ist es besagten Mengenstrom (E) zumindest teilweise und gegebenenfalls eine entsprechende Menge von (S) zunächst dem als Extraktionsmittel in Stufe (5) verwendeten Mengenstrom (J) einzuverleiben und nach Durchlaufen der Extraktionsstufe (5) der resultierenden organische Phase (L) einem bezogen auf den Gehalt an hydrophobem "Lösungsmittel" zugesetzten Teilstrom von (E) äquivalenten Teilstrom von (L) zu entnehmen und diesen dem Mengenstrom (B) zuzusetzen.

Besonders bevorzugt ist es, den besagten Teilstrom von (E) zumindest teilweise bereits der als Extraktionsmittel in der Extraktionsstufe (6) verwendeten organischen Phase (P) zuzusetzen und als Teil der resultierenden organischen Phase (R) der Extraktionsstufe (5) und anschließend als Teilmenge von (L) dem Mengenstrom (B) zuzusetzen.

Gegebenenfalls kann gemäß einer weniger allgemeinen anwendbaren Anwendungsform bei der Bildung von Mengenstrom (B) vollständig auf die Zuspeisung von hydrophobem Lösungsmittel und Hilfsamin aus anderen Quellen verzichtet werden, so daß die organische Phase (B) ausschließlich aus einer Teilmenge von (L) besteht.

Diese Ausführungsform des erfindungsgemäßen Verfahrens ist bezüglich Trennleistung und Energiebilanz besonders vorteilhaft und daher besonders bevorzugt, wenn sie angewendet werden kann.

Gegebenenfalls wird in einer weniger allgemein anwendbaren Ausführungsform die Gesamtmenge der Mengenströme (E) und (F) dem Mengenstrom (J) zugeschlagen und zunächst als Extraktionsmittel in Extraktionsstufe (5)

verwendet, so daß der Mengenstrom (B) ausschließlich aus einer Teilmenge des die Extraktionsstufe (5) verlassenden Mengenstromes (L) gebildet wird.

Im allgemeinen beträgt der Gehalt der organischen Phase (B) an aromatischem Amin je nach Trennaufgabe 25 - 60 %.

In der vorzugsweise mehrstufig betriebenen Extraktionsstufe (4) werden die organische Phase (B) und das Gemisch aus Ausgangspolyamingemisch (A) und wäßriger Phase (C) unter inniger Durchmischung einander entgegengeführt.

Bei diesem Vorgang findet in der Regel ein teilweiser Übergang von Polyamin in die organische Phase statt, gegebenenfalls im Austausch von Hilfsamin in entgegengesetzter Richtung.

Das zusammen mit der wäßrigen Phase (C) in den Extraktor (4) eingebrachte Ausgangspolyamin (A) verteilt sich auf die den Extraktor verlassende wäßrige Phase (H) und die den Extraktor verlassende organische Phase (D) (quantitative Fraktionierung).

Die mengenmäßige Aufteilung der einzelnen Komponeten des Ausgangspolyamingemisches auf die resultierende wäßrige Phase (H) und die resultierende organische Phase (D) erfolgt unter den Bedingungen des erfindungsgemäßen Verfahrens mit einer überraschend hohen Selektivität, so daß die resultierenden Produktfraktionen eine andere, unter Umständen stark von der des Ausgangspolyamingemisches abweichende Zusammensetzung aufweisen (qualitative Fraktionierung).

Beispielsweise ausgehend von den bevorzugt eingesetzten Anilinformaldehydkondensationsprodukten wurde gefunden, daß von einer in zwei oder mehreren isomeren Formen im Ausgangsgemisch enthaltenen Polyaminkomponente in der Regel die ortho-isomere(n) Form(en) in der die Trennstufe (4) verlassenden organische Phase (D) relativ angereichert ist (sind); beispielsweise 2.4'-Diamino-diphenylmethan relativ zu 4,4'-Diamino-diphenylmethan. Umgekehrt ist die resultierende wäßrige Phase (H) relativ verarmt an dem 2,4'-Isomeren, während das 4,4'-Isomere relativ angereichert ist.

Sind mehrere "ortho-Isomere" im Ausgangspolyamin vorhanden, z. B. 2,2'- und 2,4'-Diamino-diphenylmethan, dann ist das "ortho-reichere" 2,2'-Isomere in der organischen Phase (D) gegenüber dem "ortho-ärmeren" 2,4'-Isomeren stärker angereichert, welchletzteres seinerseits gegenüber dem "noch ortho-ärmeren" 4,4'-Isomeren relativ angereichert ist.

Der zuerst bei den Anilinformaldehydkondensationsprodukten der Diamino-diphenylmethanreihe gefundene An- und Abreicherungseffekt wurde rein empirischdeskriptiv mit dem Kriterium der ortho- und para-Substitution verbunden. Die davon abgeleitete Charakterisierung der Verfahrensprodukte als "orthoreich" und "orthoarm" ist dabei relativ und wurde durch den Begriff "ortho-Substitutionsgrad" ausgedrückt.

Als "ortho-Substitutionsgrad" wird dabei das Verhältnis der orthoständigen Aminogruppen-Methylengruppenrelationen, zur Gesamtzahl aller Aminogruppenrelationen definiert. Mit diesem Begriff lassen sich praktisch alle Isomerentrennungen bei den Polyaminen erfassen, die aus Arylaminen, auch substituierten, mit Carbonylverbindungen in wäßrig saurem Medium hergestellt werden.

Überraschenderweise wurde nun der gleiche An- und Abreicherungseffekt - geordnet nach ortho-Substitutionsgrad- auch für die gut charakterisierten und analytisch erfaßbaren isomeren Dreikernverbindungen aus der Anilin-Formaldehydkondensation gefunden.

Analoges gilt für die Trennung der Isomeren von Kondensationsprodukten aus Formaldehyd mit Anilin und Diaminoarylverbindungen wie Phenylendiamin oder alkylsubstituierten Phenylendiaminen.

Die bisher erwähnten Polyamingemische weisen, bedingt durch ihre Herstellung, Aminogruppen auf, die praktisch nur orthoständig und/oder paraständig zu Methylengruppen sind. Dabei werden innerhalb einer Gruppe isomerer Verbindungen in der Regel diejenigen mit dem höheren ortho-Substitutionsgrad gegenüber den Isomeren mit einem geringeren ortho-Substitutionsgrad bei der Fraktionierung in der organischen Phase (D) angereichert.

Polyamingemische insbesondere der Diphenylmethanreihe einschließlich der jeweiligen höherkernigen Homologen, die nach anderen Verfahren hergestellt werden, beispielsweise durch Nitrierung von Diphenylmethan oder Methyldiphenylmethanen und anschließende Reduktion weisen außer ortho- und paraständigen Aminogruppen herstellungsbedingt auch andere Aminogruppen-Methylengruppenrelationen auf. Für diese Polyamingemische ist das erfindungsgemäße Verfahren genauso wirksam.

Beispielsweise läßt sich aus einem Gemisch von 2- und 4-Methyldiphenylmethan durch Nitrierung und anschließende Reduktion ein Polyamingemisch herstellen, welches in der Hauptsache ein Isomerengemisch darstellt aus

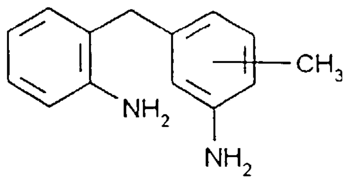

und

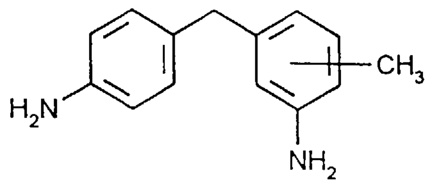

Bei der Fraktionierung solcher Gemische mit Hilfe des erfindungsgemäßen Verfahrens werden die 3.2'-Amino-Isomeren in der organischen Phase (D) gegenüber den 3,4'-Amino-Isomeren angereichert.

Das Kriterium "orthoreich" und "orthoarm" oder der ortho-Substitutionsgrad erfaßt in diesen Polyanüngemischen nicht mehr alle Isomeren und ist daher sinngemäß anzuwenden, indem anstelle der Begriffe "orthoständig" und "paraständig" eine Einteilung der Isomeren vorgenommen wird in solche mit kleinerem (= ortho-) und solche mit größerem (= para-)räumlichen Abstand der - in der Regel an unterschiedlichen Sechsringen befindlichen - Aminogruppen zur Methylenbrücke bzw. der Aminogruppen zueinander.

Eine weitere Klasse aromatischer Polyamingemische, die sich mit Hilfe des erfindungsgemäßen Verfahrens sehr wirksam fraktionieren lassen, stellen die Polyamine des Triphenylmethans und seiner höherkernigen Homologen, vorzugsweise Benzylhomologen dar, wie sie z. B. durch Nitrierung und anschließende Reduktion der entsprechenden Kohlenwasserstoffgemische hergestellt werden.

Bei der Fraktionierung technischer Polyamingemische der zuletzt genannten Substanzklassen

I. Mischkondensationsprodukte von Mono- und Diaminoarylverbindungen mit Formaldehyd bzw. allgemeinen Carbonylverbindungen,

II. Polyamingemische aus Verfahren durch Nitrierung und anschließende Reduktion von Diphenylmethan und vorzugsweise substituierten insbesondere alkylsubstituierten Diphenylmethanen und den jeweiligen Homologen und

III. Polyamingemische aus Verfahren durch Nitrierung und anschließende Reduktion von Triphenylmethan und vorzugsweise substituierten insbesondere alkyl substituierten Triphenylmethanen und den jeweiligen höherkernigen Benzylhomologen

wurde zusätzlich zur reinen Isomerentrennung eine weitere überraschende Selektivität gefunden.

Polyamingemische der genannten Substanzklasse I bis III enthalten oder können enthalten Komponenten, bei denen wengistens ein Arylkern pro Molekül mehr als eine, in der Regel zwei Aminogruppen trägt. Diese Komponenten können die bevorzugten Bestandteile des Polyamingemisches sein, ohne daß sie verfahrensbedingt mengenmäßig die Hauptprodukte sein müssen.

Zur besseren Charakterisierung solcher Komponenten wird der Begriff "Aminosubstitutionsgrad" verwendet, mit dem in erster Linie die Anzahl der Aminogruppen einer Komponente im Verhältnis zur Anzahl der Arylkerne gekennzeichnet wird.

Für Anilin und seine Kondensationsprodukte mit Formaldehyd ist dieser Ausdruck stets 1,0 ,für Phenylendiamin und seine Kondensationsprodukte stets 2,0. Für reine Mischkondensate ergeben sich für die Diphenylmethanisomeren der Wert 1,5 und für die höherkernigen Homologen Werte zwischen > 1,0 und < 2,0. Bei statistischer Verwendung des Begriffes Aminosubstitutionsgrad zur Charakterisierung von Polyamingemischen ergeben sich ebenfalls Werte zwischen 1,0 und 2,0.

Bei der Fraktionierung von Polyamingemischen mit Komponenten mit einem Aminosubstitutionsgrad > 1,0 wurde nun gefunden, daß die Komponenten mit einem höheren Aminosubstitutionsgrad in der im eigentlichen Trennschritt resultierenden wäßrigen Phase (H) relativ angereichert werden, und zwar um so stärker je größer der Aminosubstitutionsgrad ist.

Unabhängig davon ist auch hier die Trennung nach ortho-Substitutionsgrad wirksam.

Somit eröffnet das erfindungsgemäße Verfahren auch für diese Substanzklasse neue Wege, die Herstellungsform der Rohstoffe (Aminstufe) und die Verwendungsform der Endprodukte (Isocyanatstufe) durch Fraktionierung und/oder Anreicherung auf der Aminstufe und separate Weiterverarbeitung der Fraktionen zu entkoppeln, so daß eine getrennte Optimierung beider Stufen erleichert wird bis hin zur Gewinnung völlig neuer Isocyanatgemische oder erst möglich wird wo bislang geeignete Verfahren und Methoden fehlten oder wenig praktikabel sind.

Ergänzt werden diese "Leistungen" durch ein weiteres Selektivitätskriterium, welches bei der Fraktionierung technischer Polyamingemische, insbesondere solcher mit höherkernigen Homologen, gefunden wurde und die "Kernigkeit" der Polyamingemische betrifft.

Mit dem Begriff "Kernigkeit" wird primär die Anzahl der Aryleinheiten einer Komponente eines aromatischen Polyamingemisches ausgedrückt. Im weiteren Sinne wird der Begriff der Kernigkeit dafür verwendet, um für ein aus zahlreichen Komponenten, mit einer individuellen exakten Kernigkeit, bestehendes Polyamingemisch statistisch eine Kernigkeit des Gesamtgemisches ausdrücken.

Besonders überraschenderweise wurde nun bei der Fraktionierung von Polyamingemischen mit höherkernigen Anteilen, insbesondere bei der Fraktionierung technischer Gemische von Anilin-Formaldehydkondensaten, gefunden, daß sich die höherkernigen Komponenten in der die Fraktionierungsstufe verlassenden organischen Phase gezielt sowohl relativ anreichern als auch relativ abreichern lassen, in Abhängigkeit von der Molarität der wäßrigen Phase in der Extraktionsstufe (4).

Eine hohe Molarität der wäßrigen Phase in (4) innerhalb des angegebenen Molaritätsbereiches führt zu einer relativen Abreicherung höherkerniger Komponenten in der organischen Phase (D) und dementsprechend zu einer relativen Anreicherung in der wäßrigen Phase (H).

Eine niedrige Molarität der wäßrigen Phase in (4) innerhalb des angegebenen Molaritätsbereichs führt zu einer relativen Anreicherung höherkerniger Komponenten in der organischen Phase (D).

Der überraschende Befund kann dahingehend erweitert und präzisiert werden, daß die relative An- und Abreicherung auch innerhalb der höherkernigen Homologen untereinander stattfindet. Werden beispielsweise in einem technischen Gemisch des Diamino-diphenylmethans in der einen Fraktion die Dreikernkomponenten gegenüber den Zweikernkomponenten relativ an- oder abgereichert, wird auch eine relative An- oder Abreicherung von Vierkernkomponenten gegenüber Dreikernkomponenten, d.h. eine noch stärkere relative An- oder Abreicherung, gefunden, desgleichen von Fünfkernkomponenten gegenüber Vierkernkomponenten usw.

Daraus und aus der gleichzeitig und stets im Sinne einer relativen Verstärkung des "ortho-Substitutionsgrades" in der organischen Phase (D) ablaufenden Isomerentrennung und aus der Möglichkeit, mit einzelnen Produktfraktionen die erfindungsgemäße Trennung, gegebenenfalls mit veränderten Verfahrensparametern zu wiederholen, ergeben sich zahlreiche Möglichkeiten, ausgehend von bekannten und gut zugänglichen Polyamingemischen über das erfindungsgemäße Verfahren zu weniger gut zugänglichen oder völlig neuen, weil nach dem Stand der Technik bislang unzugänglichen, Polyaminen und damit Polyisocyanaten zu gelangen. Das gilt besonders für Produkte, der Diamino- und Diisocyanato-diphenylmethanreihe und ganz besonders für Polyamin- und Polyisocyanatgemische mit einem extrem hohen Anteil an höherkernigen Komponenten.

Die An- und bzw. Abreicherung wird in der Regel effektiver mit steigendem Protonierungsgrad in der wäßrigen Phase der Trennstufe.

Darüberhinaus erweist sich das erfindungsgemäße Verfahren als von allgemeiner Wirksamkeit auch auf andere strukturähnliche Polyamine.

So können beispielsweise in den bereits erwähnten Polyamingemischen, die durch Nitrierung von Di- und Polyarylmethanen und anschließender Reduktion gewonnen werden, auch Monoaminopolyarylmethanverbindungen oder Komponenten enthalten sein, bei denen eine oder mehrere Methylengruppen durch Nebenreaktionen in Keto- und/oder Hydroxymethylengruppen und damit in unerwünschte Nebenprodukte umgewandelt worden sind.

Bei der Kondensation von Arylaminen mit Carbonylverbindungen können zahlreiche unvollständig umgelagerte Zwischenverbindungen und Nebenprodukte auftreten.

Die meisten dieser Verbindungen unterliegen in der Regel bei der Fraktionierung der sie enthaltenden Polyamingemische einer Anreicherung in einer der resultierenden Fraktionen, so daß der Effekt zur Abtrennung und Fraktionierung genutzt werden kann.

Gegebenenfalls können derartige Produkte auf diesem Wege an- oder abgereichert werden oder als gezielt hergestellte Polyamingemische, wie z. B. Polyaminobenzophenone oder Aminobenzylarylamingemische ihrerseits fraktioniert werden.

Die die Extraktionsstufe (4) verlassende, organische Phase (D) enthält unter anderem noch geringe Mengen an Säure, im allgemeinen und in Abhängigkeit von den Verfahrensparametern in der Extraktionsstufe (4) zwischen 0,01 und 0,5 Gew.-%, die vorteilhaft vor der destillativen Aufarbeitung des Mengenstromes (D) entfernt werden.

Im einfachsten Falle geschieht dies durch Neutralisation mit überschüssigen, verdünnten wäßrigen Basen, beispielsweise verdünnter Natronlauge. Bevorzugt wird jedoch das Herauswaschen der Säure bzw. ihrer Aminsalze

aus der organischen Phase mit Wasser, so daß gegebenenfalls nur noch verbleibende Spuren durch Kontakt mit verdünnter Natronlauge oder mit Hilfe eines Ionentauschers entfernt werden.

Das eingesetzte Waschwasser wird unter Einschaltung eines Wasserverdampfers dem wäßrigen Säurekreislauf entzogen und diesem nach Durchlaufen der Waschstufe(n) mitsamt der Säure an verfahrensmäßig geeigneter Stelle wieder zugesetzt.

Die organische Phase (D) bzw. (D') wird, gegebenenfalls nach Durchlaufen der Säurewaschstufe (7.0), in die mindestens zweistufige Destillationsstufe (7.1), (7.2) überführt.

In der ersten Destillationsstufe (7.1) wird ein Destillat (E) abgetrennt, welches die Hauptmenge, vorzugsweise nahezu die Gesamtmenge des in (D) bzw. (D') enthaltenen, hydrophobem Lösungsmittels gegebenenfalls neben einem Teil des in ihr enthaltenen Hilfsamins umfaßt.

In der letzten Destillationsstufe (7.2) wird das verbleibende Hilfsamin, gegebenenfalls neben der Restmenge des hydrophobem Lösungsmittels, als Destillat (F) von dem als Destillationssumpf anfallenden und im Verfahrensprodukttank (10) gesammelten ersten Teil produkt (G) abgetrennt.

Das entsprechende zweite Verfahrensprodukt befindet sich in der die Extraktionsstufe (4) verlassenden wäßrigen Phase (H).

In einer mehrstufig wirkenden, vorzugsweise bei 80 - 100°C betriebenen Extraktionsstufe (5) wird aus der wäßrigen Phase (H), gegebenenfalls nach Zugabe von Wasser zur Absenkung der Molarität und gegebenenfalls nach Zugabe von Hilfsamin zur Absenkung des Protonierungsgrades, das zweite Teilprodukt im Austausch gegen Hilfsamin extrahiert und dabei in die organische Phase (L) überführt.

Die Molarität der in (5) eingesetzten wäßrigen Phase ist vorzugsweise < 2,5.

Der Protonierungsgrad der in (5) eingesetzten wäßrigen Phase ist vorzugsweise < 60 %.

Als Extraktionsmittel (J) dient ein Gemisch aus hydrophobem Lösungsmittel und Hilfsamin, welches im wesentlichen aus der die nachfolgende Extraktionsstufe (6) verlassenden organischen Phase (R), gegebenenfalls Destillat (M), gegebenenfalls einer Teilmenge von (E) und in der Regel einer Teilmenge des Mengenstromes (S) gebildet wird.

Das Gewichtsverhältnis von Hilfsamin zu Lösungsmittel liegt in (J) im allgemeinen zwischen 0,5 : 1 und 3 : 1, vorzugsweise zwischen 1 : 1 und 2 : 1.

Das Gewichtsverhältnis von Extraktionsmittel (J) zu wäßriger Phase liegt im allgemeinen zwischen 0,3 : 1 und 3 : 1, vorzugsweise zwischen von 0,7 : 1 und 2 : 1.

Die in (5) resultierende organische Phase (L) bzw. (L') wird, gegebenenfalls nach Durchlaufen der Waschstufe (8.0) und/oder gegebenenfalls nach Entfernen von Säurespuren mit verdünnter Natronlauge, den Destillationsstufen (8.1) und (8.2) zugeführt.

In der Destillationsstufe (8.1), (8.2) erfolgt die destillative Abtrennung des Destillationsrückstandes (O), der als zweites Teilprodukt in den Verfahrensprodukttank (11) gesammelt wird.

Die Aufarbeitungsstufe (8) besteht im allgemeinen aus einer wenigstens zweistufigen Mehrstufendestillation (8.1) und (8.2), in deren Stufe (8.1) ein gegenüber dem Zulaufprodukt (L) bzw. (L') von Polyamin befreites und an Hilfsamin stark verarmtes hydrophobes Lösungsmittel als Destillat (M) gewonnen wird.

Der Gehalt von (M) an Hilfsamin beträgt im allgemeinen < 30 %, vorzugsweise < 20 %; gegebenenfalls wird in (8.1) ein von Hilfsamin befreites hydrophobes Lösungsmittel als Destillat gewonnen.

Das Destillat (M) wird zumindest teilweise, gegebenenfalls auch nach Zugabe von einer Teilmenge von (E) aus der Destillationsstufe (7.1), eingesetzt in der Extraktionsstufe (6) als organische Phase (P) zur Gegenstromextraktion von wenigstens einer Teilmenge der in der Extraktionsstufe (5) resultierenden wäßrigen Phase (K), welche der Extraktionsstufe (6) direkt und/oder indirekt über eine zwischengeschaltete Destillationsstufe (9) zumindest teilweise zugeführt wird.

Bei dem in der Extraktionsstufe (6) ablaufenden Extraktionsvorgang wird der wäßrigen Phase Hilfsamin und gegebenenfalls noch vorhandenes Polyamin entzogen, so daß die resultierende wäßrige Phase (Q) stets einen höheren Protonierungsgrad aufweist als die wäßrige Phase (K) aus der Extraktionsstufe (5).

Der Protonierungsgrad von (Q) beträgt im allgemeinen > 50 %, vorzugsweise > 60 % und kann bei Verwendung von reinem hydrophoben Lösungsmittel als (P) bis zu 100 % betragen.

Es ist ein Kennzeichen des erfindungsgemäßen Verfahrens, daß die wiederverwendete wäßrige Phase (C) einen höheren Protonierungsgrad aufweist als die die Extraktionsstufe (5) verlassende wäßrige Phase (K).

Für Protonierungsgrade, die zwar höher als derjenige von (K) aber < 100 % betragen, kann das erreicht werden, indem entweder die gesamte wäßrige Phase die Extraktionsstufe (6) durchläuft, oder nur eine entsprechende Teilmenge, die gegebenenfalls mit einem Protonierungsgrad von 100 % durch Vermischen mit dem Rest den gegenüber (K) erhöhten Protonierungsgrad von (C) ergibt.

Ein Protonierungsgrad von praktisch 100 % in der wäßrigen Phase (C) kann nur erreicht werden, wenn die gesamte wäßrige Phase wenigstens einmal über die Extraktionsstufe (6) läuft und dabei mit praktisch reinem hydrophoben Lösungsmittel extrahiert wird.

Da die wäßrige Phase (C) erfindungsgemäß einen höheren Protonierungsgrad aufweist als den in der wäßrigen Eingangsphase zur Extraktionsstufe (5) bevorzugten Protonierungsgrad entspricht, kann es erforderlich und vorteilhaft sein, durch Zugabe von Hilfsamin und/oder Polyamin an geeigneter Stelle einen solchen bevorzugten Protonierungsgrad in der wäßrigen Eingangsphase zur Extraktionsstufe (5) einzustellen.

Geeignete Stellen für die Aminzufuhr sind die wäßrige Phase unmittelbar vor Eintritt in die Extraktionsstufe (5) unter Verwendung von Hilfsamin oder an vorgelagerter Stelle, vorzugsweise vor Eintritt der wäßrigen Phase in die Extraktionsstufe (3) beispielsweise durch direkte Zugabe oder über eine vorgeschaltete Extraktionsstufe (2).

Ein weiterer wichtiger Parameter, der zurückgeführten und wiederverwendeten wäßrigen Phase (C) ist die Molarität. Erfindungsgemäß kann die Molarität von (C) in einem weiteren Bereich variiert werden in Abhängigkeit von der jeweiligen Trennaufgabe.

Grundsätzlich kann die in Verfahrensstufe (4) resultierende wäßrige Phase (H) direkt der Extraktionsstufe (5) zugeführt werden. Da aber der obere, für bestimmte Trennaufgaben durchaus bevorzugte Molaritätsbereich der Extraktionsstufe (4) oberhalb des bevorzugten Molaritätsbereichs der Extraktionsstufe (5) liegt, ist es eine bevorzugte Ausführungsform des erfindungsgemäßen Verfahrens, die Molaritäten in den verschiedenen Extraktionsstufen dadurch zu entkoppeln, daß dem in sich geschlossenen System der wäßrigen Phase gegebenenfalls an geeigneter Stelle destillativ Wasser entzogen und an anderer geeigneter Stelle wieder zugesetzt wird.

Mit Hilfe einer Wasserdestillationsstufe (9) wird der wäßrigen, die Säure enthaltende Phase, oder einem Teilstrom der wäßrigen Phase, vorzugsweise nach Verlassen der Extraktionsstufe (5) und vor der Wiederverwendung am Prozessbeginn Wasser entzogen, welches spätestens vor Eintritt der wäßrigen Phase in die Extraktionsstufe (5) insgesamt (Y) oder in Teilmengen an einer oder mehreren Stellen wieder zugesetzt wird.

Ob der Entzug von Wasser in (9) vor oder nach Durchlaufen der Extraktionsstufe (6) erfolgt, ist für die erfindungsgemäße Durchführung ohne Belang.

Das in (9) entzogene Wasser (X) kann gleichzeitig zum Betreiben der Waschstufen (7.0) und/oder (8.0) genutzt werden.

Die Wassermenge (X) kann auch überwiegend oder ausschließlich zum Betreiben der Waschstufen (7.0) und/oder (8.0) entzogen werden.

Mit dieser ersten Variante des erfindungsgemäßen Verfahrens lassen sich beträchtliche Trennleistungen bei der Fraktionierung von Polyamingemischen erzielen und zahlreiche Trennprobleme zufriedenstellend lösen.

Insbesondere in der zweiten Polyaminfraktion (O) kann die relative Anreicherung der in dieser Fraktion bevorzugt enthaltenen Komponenten gezielt variiert und maximiert werden.

Der in der ersten Polyaminfraktion (G) verbleibende Anteil dieser Komponenten kann jedoch gemäß dieser ersten Variante nicht in gleicher Weise minimiert werden, sondern variabel nur bis zu einem Gehalt relativ abgereichert werden, dessen untere Grenze abhängt von dem für die jeweiligen Verfahrensparameter charakteristischen Verteilungsgleichgewichte der Polyaminkomponenten von (A) zwischen der wäßrigen Phase beim Eintritt in den Extraktor (4) und der organischen Phase (D) beim Verlassen des Extraktors (4).

Vorteilhafter und als Ausführungsform bevorzugt ist eine zweite Variante des erfindungsgemäßen Verfahrens, bei der sich zusätzlich auch in der ersten Polyaminfraktion (G) die relative Anreicherung der in dieser Fraktion bevorzugt enthaltenen Komponenten beträchtlich steigern und gezielt variieren läßt, indem die in der Extraktionsstufe (4) anfallende organische Phase (D) zumindest teilweise in einer aus der Sicht der Phase (D) zwischengeschalteten Extraktionsstufe (3) mit einer wäßrigen Phase extrahiert wird, die im vorliegenden Fall der Variante 2 im wesentlichen aus zumindest einer Teilmenge des Mengenstrom (C) besteht.

Aus formalen Gründen wird die der Extraktionsstufe (3) zugeführte organische Phase als Mengenstrom (T) bezeichnet, auch wenn sie gegebenenfalls wie im vorliegenden Fall beispielhaft ausgeführt zumindest in der Zusammensetzung, vorzugsweise aber auch in der Menge mit Mengenstrom (D) übereinstimmt.

Bereits bei der einstufigen Durchführung der Extraktionsstufe (3), beispielsweise als Mischer-Scheidereinheit, kommt es in der resultierenden organischen Phase (V) in Abhängigkeit von Art und insbesondere Menge der eingesetzten wäßrigen Phase zu einer deutlichen weiteren relativen Anreicherung der bereits in (D) gegenüber Ausgangspolyamin (A) angereicherten Komponenten verbunden mit einer Abnahme des Polyamingehaltes in der organischen Phase (V). Vorzugsweise wird jedoch auch die zwischengeschaltete Extraktionsstufe (3) als mehrstufig wirkender und im Gegenstrom betriebener Extraktor wegen der besseren Effektivität ausgeführt.

Die in der Extraktionsstufe (3) anfallende wäßrige Phase (U) enthält die entsprechende andere Fraktion des mit Mengenstrom (T) eingebrachten Polyamin, in der die in (V) angereicherten Komponenten entsprechend abgereichert sind. Das ereichbare Ausmaß der relativen Abreicherung, d.h. die Zusammensetzung des in (U) enthaltenen Polyamins wird unter den jeweiligen Verfahrensbedingungen der mehrstufig wirkenden Extraktionsstufe (3) kontrolliert durch das qualitative und quantitative Verteilungsgleichgewicht zwischen der zugeführten organischen Phase (T) und der abgeführten wäßrigen Phase (U).

Die Molarität der wäßrigen Phase in der Extraktionsstufe (3) liegt je nach Trennaufgabe höher oder auf gleicher Höhe oder niedriger bezogen auf die Molarität in der aus Sicht der wäßrigen Phase nachgeschalteten Extraktionsstufe

(4) und wird durch Zugabe oder Entzug von Wasser an geeigneter Stelle geregelt.

Die in Stufe (3) resultierende wäßrige Phase (U) wird gegebenenfalls nach Zugabe von Wasser zusammen mit den gegebenenfalls vorhandenen Rest von (C) der Extraktionsstufe (4) zugeführt.

Die in Stufe (3) resultierende organische Phase (V) wird zusammen mit dem gegebenenfalls vorhandenen Rest von (D) der Aufarbeitungsstufe (7) zugeführt zur Gewinnung der Polyaminfraktion (G).

Mit der zweiten Variante des erfindungsgemäßen Verfahrens läßt sich die relative Anreicherung in beiden resultierenden Polyaminfraktionen gezielt variieren und maximieren. Neben dieser in qualitativer Hinsicht großen Vielseitigkeit und Leistungsfähigkeit bietet die zweite Verfahrensvariante zumindest für die zweite Polyaminfraktion (O) auch eine energetisch günstige Ausführungsform.

Der mit Gewinnung der ersten Polyaminfraktion (G) verbundene Energieaufwand steigt dagegen relativ um so stärker, je geringer der mengenmäßige Anteil von (G), bezogen auf eingesetztes Polyamingemisch (A) ist, weil der verbleibende Gehalt an Polyamin (G) in der destillativ aufzuarbeitenden organischen Phase (D) bzw. (V) entsprechend immer kleiner wird.

Der Effekt kommt besonders dann zum Tragen, wenn die mit (G) abgetrennten Komponenten im Ausgangsgemisch (A) nur in geringer Konzentration enthalten sind und/oder relativ hoch in der Fraktion (G) angereichert werden, z.B. bei der erfindungsgemäßen Auftrennung von Polyamingemischen der Diphenylmethanreihe.

Eine in dieser Hinsicht verbesserte Ausführungsform stellt die dritte Variante des erfindungsgemäßen Verfahrens dar. Ausgehend von der ersten Variante, wird diese dahingehend erweitert, daß die die Verfahrensstufe (4) verlassende organische Phase (D) geteilt wird in einen Teilstrom (D'), der weiterhin mit dem Ziel der Gewinnung der Polyaminfraktion (G) der Aufarbeitungsstufe (7) zugeführt wird und einen zweiten Teilstrom, der einer vorgeschalteten Extraktionsstufe (2) zugeführt wird.

Aus formalen Gründen wird die der vorgeschalteten Extraktionsstufe (2) zugeführte organische Phase als Mengenstrom (W) bezeichnet, auch wenn sie gegebenenfalls wie im vorliegenden Fall beispielhaft ausgeführt in der Zusammensetzung mit Mengenstrom (D) übereinstimmt.

Bei der Extraktionsstufe (2) handelt es sich in der Regel um einen mehrstufig wirkenden und im Gegenstrom betriebenen Extraktor, in welchem die zugeführte organische Phase (W) mit zumindest einer Teilmenge der zur Wiederverwendung zur Verfügung stehenden wässrigen Phase (C) extrahiert wird.

Der dem Extraktor (2) zugeführte Mengenstrom (W) wird dabei so bemessen, daß bei der Umsetzung mit Mengenstrom (C) ein möglichst weitgehender, vorzugsweise praktisch quantitativer Übergang der in der organischen Phase (W) enthaltenen Polyamine in die den Extraktor (2) verlassende wäßrige Phase (Z) erfolgt.

Eine erhöhte Molarität in der in Stufe (2) eingesetzten wäßrigen Phase, resultierend aus Verfahrensstufe (9) des erfindungsgemäßen Verfahrens, begünstigt und erleichert den Übergang von Polyamin aus der organischen Phase (W) in die wäßrige Phase (Z).

Ein erhöhter Protonierungsgrad der in Stufe (2) eingesetzten wäßrigen Phase, resultierend aus Verfahrensstufe (6) des erfindungsgemäßen Verfahrens, begünstigt und erleichert ebenfalls den Übergang von Polyamin aus der organischen Phase (W) in die wäßrige Phase (Z).

Der Restgehalt an Polyamin in der die Verfahrensstufe (2) verlassenden organischen Phase liegt im allgemeinen bei < 5 Gew.-%, vorzugsweise bei < 1 Gew.-%.

Im übrigen richtet sich der zulässige Höchstgehalt an Amin und insbesondere der Gehalt an Polyamin nach den aus der jeweiligen Trennaufgabe resultierenden qualitativen Anforderungen an die Verfahrensprodukte, d.h. die Qualität der Trennung, im Falle der Variante 3 insbesondere an das Verfahrensteilprodukt (O). Die Einhaltung des für die Qualität von (O) relevanten Gehaltes an Polyamin wird im Rahmen der technischen Gegebenheiten unter Ausschöpfung der zur Verfügung stehenden wäßrigen Phase über die Bemessung des Teilmengenstromes (W) kontrolliert.

Dabei kommt es dem Verfahren und insbesondere der Extraktionsstufe (2) zustatten, daß die für den Einsatz in Stufe (2) zur Verfügung stehende wäßrige Phase (Mengenstrom C) um so größer ist, je größer der Anteil der zweiten Polyaminfraktion (O) ist und je kleiner demzufolge der Anteil der ersten Polyaminfraktion (G) ist. Eine kleine Polyaminfraktion (G) bedeutet in der Regel eine niedrige Polyaminkonzentration in der organischen Phase (D) und hohen Energieaufwand bei der Aufarbeitung einer solchen Phase.

Durch die erfindungsgemäße Variante 3 kann gegenüber Variante 1 insbesondere der Energieaufwand für die Isolierung der ersten Polyaminfraktion (G) reduziert werden.

Der Beitrag der Verfahrensstufe (2) im Rahmen der Variante 3 zur Verbesserung des erfindungsgemäßen Verfahrens besteht darin, daß die destillative Aufarbeitung (6) zur Gewinnung der ersten Polyaminfraktion (G) anstelle des Gesamtstromes (D) mit einer relativ niedrigen und damit energetisch ungünstigen Konzentration an Polyamin nur ein Teilstrom (D') mit entsprechend erhöhter und damit energetisch günstigerer Konzentration resultiert (quantitative Anreicherung), während aus dein anderen Teilstrom von (D) eine an geeigneter Stelle als Extraktionsmittel verwendbare organische Phase ohne Destillation gewonnen wird.

Die von Polyamin weitgehend befreite, die Verfahrensstufe (2) verlassende organische Phase wird der Extraktionsstufe (4) zugeführt und als Extraktionsmittel eingesetzt, in der Regel durch Vermischen mit Mengenstrom (B)

und Zugabe zu der aus der Sicht der organischen Phase (B) ersten Stufe des Extraktors (4).

In Abhängigkeit von einem gegebenenfalls vorhandenen Restgehalt an Polyamin und mit Rücksicht auf die Qualität der zweiten Polyaminfrakion (O) erfolgt die Zugabe der in (2) gewonnenen organischen Phase gegebenenfalls zu einer aus Sicht der organischen Phase (B) späteren, gegebenenfalls zur letzten Stufe der mehrstufig arbeitenden Extraktionsstufe (4).

Die die Verfahrensstufe (2) verlassende wäßrige Phase enthält neben der in Form ihrer Ammoniumsalze vorliegenden Säure noch Hilfsamin und Polyamin, letzteres mit einer Zusammensetzung, die weitgehend dem Polyamin in der zugeführten organischen Phase (W) entspricht.

Im Falle der Variante 3 des erfindungsgemäßen Verfahrens wird der Mengenstrom direkt der Verfahrensstufe (4) zugeführt, gegebenenfalls nach Zugabe von Wasser aus Mengenstrom (Y) und/oder weiterer wäßriger Phase aus Mengenstrom (C).

Da die in der wäßrigen Phase enthaltene Polyaminfraktion in der Regel eine höhere relative (qualitative) Anreicherung im Sinne der ersten Polyaminfraktion (G) bezogen auf das Ausgangspolyamin (A) aufweist, resultiert für die der Extraktionsstufe (4) zugeführten wäßrigen Phase nach Zugabe von Ausgangspolyamin (A) ein gegenüber diesem in Abhängigkeit vom Mengenverhältnis "angereichertes" Mischpolyamin. Infolge des Verteilungsgleichgewichtes zwischen zugeführter wäßriger und resultierender organischer Phase (D) ergibt sich daraus für Variante 3 auch ein begrenzter qualitativer Anreicherungseffekt für die erste Polyaminfraktion (G).

In einer weiteren Variante 4 des erfindungsgemäßen Verfahrens werden die technischen Maßnahmen der vorangegangenen Variante zusammengefaßt und miteinander kombiniert.

Im einfachsten Fall werden die Extraktionsstufe (2) und (3) hinzugefügt und jede für sich mit einem Teilstrom von (C) und einem Teilstrom von (D), der in diesem Fall in 3 Teilströme aufgeteilt wird, in der beschriebenen Weise durchgeführt.

Vorteilhafter ist es, als organische Phase (W) in Extraktionsstufe (2) einen Teilstrom der qualitativ hochangereicherten, quantitativ weniger konzentrierten Polyaminfraktion, enthaltenden im Mengenstroms (V), einzusetzen.

Bevorzugt wird die Variante 4 so durchgeführt, daß als organische Phase (W) in der Extraktionsstufe (2) ein Teilstrom von (D) und/oder vorzugsweise ein Teilstrom von (V) eingesetzt wird und die in Stufe (2) resultierende wäßrige Phase (Z) zumindest teilweise, vorzugsweise insgesamt der Extraktionsstufe (3) zugeführt und gegebenenfalls unter Zugabe von weiterer wäßriger Phase aus Mengenstrom (C) und gegebenenfalls von Hilfsamin in (3) eingesetzt wird. Dabei wird ihr unter inniger Durchmischung in mehreren Stufen die organische Phase (T) mit ihrem Gehalt an im gleichen Sinne angereicherten Polyamin entgegengeführt, gegebenenfalls wir die organische Phase (T) verstärkt durch Zugabe eines Teilstromes der in der Extraktionsstufe (2) resultierenden organischen Phase zu (T).

Durch diese Maßnahme kommt es in der in (3) resultierenden organischen Phase (V) zu einer weiteren Steigerung des qualitativen Anreicherungseffektes. In quantitativer Hinsicht kann dieses Ergebnis durch Bemessung und Aufteilung der Mengenströme bei einem relativ hohen und damit energetisch günstigen Polyamingehalt in den in (3) resultierenden Phasen, insbesondere in der organischen Phase (V) erreicht werden.

Die Rückkopplung des Anreicherungseffektes in (V) über den Mengenstrom (W) als Teilstrom von (V) und über die wäßrige Phase (Z) wirkt dabei selbstverstärkend.

Durch die erfindungsgemäße Ausführung und Verschaltung der Extraktionsstufen (2) bis (4) in Variante (4) mit Verfahrenskriterien wie Disproportionierung anstelle von fraktionierter Extraktion in Stufe (3), mit Selbstverstärkung durch Verschaltung mit Extraktionsstufe (2) und Wiedergewinnung von Extraktionsmittel in Stufe (2) ohne Destillation für den Einsatz in der Verfahrensstufe (4) und gegebenenfalls in (3) resultiert ein Maximum an qualitativer Trennleistung, die in Kombination mit der Variation der Molarität der wäßrigen Phasen in den Stufen (2) bis (4) zu einer großen Anwendungsbreite des erfindungsgemäßen Verfahrens führt.

## Beispiel 1

Ausgangspolyamingemisch (A) (1,600 kg/h) wird mit dem im wesentlichen aus Polyamingemisch, Anilin, Chlorwasserstoff und Wasser bestehenden Mengenstrom (C) (5,050 kg/h) vermischt.

Die resultierende wäßrige Phase (Mengenströme A+C) hat folgende durchschnittliche Zusammensetzung:

| | |
|---|---|
| Mengenstrom (A)+(C) ( 6,650 kg/h ) | 24,7 % Polyarylamin |
| | 15,1 % Anilin |
| | 6,0 % Chlorwasserstoff |
| | 54,2 % Wasser. |

und wird in einem mehrstufig wirkenden Extraktor (4) bei 90°C dem organischen Mengenstrom (B) entgegengeführt,

der die folgende Zusammensetzung hat:

| | |
|---|---|
| Mengenstrom (B) ( 2,700 kg/h ) | 37,3 % Anilin<br>62,1 % Xylol<br>≤0,6 % Wasser. |

Die dabei resultierende, die Extraktionsstufe (4) verlassende, organische Phase (Mengenstrom D) hat folgende durchschnittliche Zusammensetzung:

| | |
|---|---|
| Mengenstrom (D) ( 3,009 kg/h ) | 23,4 % Polyarylamin<br>19,2 % Anilin<br>55,7 % Xylol<br>≤ 0,1 % Chlorwasserstoff<br>≤1,6 % Wasser. |

Mengenstrom (D) wird in der Waschstufe (7.0) mit Wasser (0,300 kg/h) als Teil von Mengenstrom (X) gewaschen. Das Waschwasser wird über Mengenstrom (Y) dem wässrigen Mengenstrom (H) zugeführt.
Zur Sicherheit wird der gewaschene Mengenstrom (D) mit verdünnter Natronlauge gewaschen. Die wäßrige Phase wird als Abwasser entsorgt.
Der gewaschene und von Säureresten befreite Mengenstrom (D) wird in einer ersten Destillationsstufe (7.1) von Xylol und einem Teil des Anilins befreit. Aus dem Destillat Mengenstrom (E) wird ein Teil des Wassers mechanisch abgetrennt und Mengenstrom (X) zugeschlagen.
In der zweiten Destillationsstufe (7.2) wird aus der Sumpfphase von (7,1) das restliche Anilin (Mengenstrom F) abgetrennt. Als Destillationssumpf von Stufe (7.2) verbleibt ein Polyamingemisch, welches als Mengenstrom (G) mit 0,704 kg/h in Tank (10) gesammelt wird.
Die Destillatströme (E) und (F) bilden zusammen mit einer Teilmenge von 0,430 kg/h aus Mengenstrom (N) den Mengenstrom (B).
Die die Extraktionsstufe (4) verlassende wäßrige Phase (H) hat folgende durchschnittliche Zusammensetzung:

| | |
|---|---|
| Mengenstrom (H) ( 6,341 kg/h ) | 14,8 % Polyarylamin<br>22,6 % Anilin<br>6,2 % Chlorwasserstoff<br>56,4 % Wasser. |

Nach Zumischen von Mengenstrom (Y) (0,908 gk/h) wird die resultierende wäßrige Phase in einem mehrstufig wirkenden Extraktor (5) bei 90°C mit einer organischen Phase (Mengenstrom J) im Gegenstrom extrahiert.

| | |
|---|---|
| Mengenstrom (J) ( 4,200 kg/h ) | 52,1 % Anilin<br>47,3 % Xylol<br>≤0,6 % Wasser |

Die dabei resultierende, die Extraktionsstufe (5) verlassende, organische Phase (Mengenstrom L) hat folgende Zusammensetzung:

| | |
|---|---|
| Mengenstrom (L) ( 4,940 kg/h ) | 18,1 % Polyarylamin<br>39,0 % Anilin<br>40,2 % Xylol<br>≤0,1 % Chlorwasserstoff<br>≤1,6 % Wasser. |

Mengenstrom (L) wird in der Waschstufe (8.0) mit Wasser (0,600 kg/h) als Teil von Mengenstrom (X) gewaschen. Das Waschwasser wird über Mengenstrom (Y) dem wäßrigen Mengenstrom (H) zugeführt.

Zur Sicherheit wird der gewaschene Mengenstrom (L) mit verdünnter Natronlauge gewaschen. Die wäßrige Phase wird als Abwasser entsorgt.

Der gewaschene und von Säureresten befreite Mengenstrom (L) wird anschließend in einer ersten Destillationsstufe (8.1) aufgetrennt in eine erste Destillatfraktion (M), die praktisch aus Xylol mit einem Anilingehalt von ≤1% besteht
Mengenstrom (M) ( 2,033 kg/h )
und in einen Destillationssumpf, welcher in einem zweiten Destillationsschritt aufgetrennt wird in eine zweite Destillatfraktion (N), die praktisch aus Anilin besteht
Mengenstrom (N) (1,953 kg/h)
und in einen aus Polyamin bestehenden Destillationsrückstand (O).
Mengenstrom (O) (0,896 kg/h)

Die Polyaminfraktion (O) wird als zweites Teilprodukt im Tank (11) gesammelt.

Die den Extraktor (5) verlassende wäßrige Phase (K) hat folgende durchschnittliche Zusammensetzung

| | |
|---|---|
| Mengenstrom (K) ( 6,509 kg/h ) | 0,6 % Polyarylamin<br>25,3 % Anilin<br>6,1 % Chlorwasserstoff<br>68,0 % Wasser |

und wird in einem weiteren mehrstufig wirkenden Extraktor (6) bei 90°C einer organischen Phase(P) entgegengeführt. (P) ist im vorliegenden Fall identisch nach Menge und Zusammensetzung mit der ersten Destillatfraktion (M) aus der Destillationsstufe (8.1).

Bei diesen Vorgang resultiert als organische Phase der Mengenstrom (R)

| | |
|---|---|
| Mengenstrom (R) ( 2,677 kg/h ) | ≤0,2 % Polyarylamin<br>24,8 % Anilin<br>74,2 % Xylol<br><1,0 % Wasser. |

Aus Mengenstrom (R) wird durch Zumischen eines Teilstromes von (N) (1,523 kg/h) der Mengenstrom (J) gebildet.

Die in (6) resultierende wäßrige Phase (Q) hat folgende durchschnittliche Zusammensetzung:

| | |
|---|---|
| Mengenstrom (Q) ( 5,865 kg/h ) | 0,6 % Polyarylamin<br>17,1 % Anilin<br>6,8 % Chlorwasserstoff<br>75,5 % Wasser. |

In einer nachfolgenden Destillationsstufe (9) wird der wäßrigen Phase (Q) destillativ Wasser in Form des Mengenstromes (X) entzogen.
Mengenstrom (X) (0,815 kg/h)

Das in (9) abdestillierte Wasser wird zusammen mit dem in den Destillationsstufen (7.1) und (8.1) mechanisch abgetrennten Wasser zum Waschen der organischen Phase (D) in (7.0) und der organischen Phase (L) in (8.0) eingesetzt. Die resultierenden Waschwässer werden in Mengenstrom (Y) vereinigt und dem wäßrigen Mengenstrom (H) zugeschlagen vor dessen Eintritt in die Extraktionsstufe (5).

Die in der Destillationsstufe (9) resultierende wäßrige Phase (S) wird der Extraktionsstufe (4) zugeführt und ist im vorliegenden Fall nach Menge und Zusammensetzung identisch mit dem dort eingesetzten wäßrigen Mengenstrom (C).

| Polyarylamin | A | G | N |
|---|---|---|---|
| GC: | [Gew.-%] | [Gew.-%] | [Gew.-%] |
| 2,2'-Diamino-diphenylmethan | 0,50 | 1,15 | - |
| 2,4'-Diamino-diphenylmethan | 11,60 | 25,45 | 0,70 |
| 4,4'-Diamino-diphenylmethan | 51,50 | 35,50 | 64,10 |
| N-Methyl-4,4'-diamino-diphenylmethan | 0,40 | 0,90 | <0,10 |
| Σ-Diamino-diphenylmethane | 64,00 | 63,00 | 64,80 |
| Σ-Mehrkernpolyamine | 36,00 | 37,00 | 35,20 |
| Mengenverteilung | 100 % | 44,0 % | 56,0 % |

## Patentansprüche

**1.** Verfahren zur Fraktionierung und Reinigung von aromatischen Polyamingemischen der Diphenylmethanreihe, dadurch gekennnzeichnet, daß man

a) das Polyaminausgangsgemisch (A) in einem zweiphasigen System, bestehend aus (i) einer hydrophoben Lösungsmittelphase (B), die im wesentlichen aus hydrophobem Lösungsmittel besteht, und (ii) einer wäßrigen Phase (C), bestehend im wesentlichen aus Wasser, einer starken Säure und zumindest teilweise in der Salzform vorliegendem Hilfsamin, welches in Wasser praktisch unlöslich ist und unter Normaldruck einem mindestens 20°C unter dem Siedepunkt der am niedrigsten siedenden Komponente des Ausgangsgemisches und mindestens 20°C oberhalb des Siedepunktes des Lösungsmittels liegenden Siedepunkt aufweist, unter Zuhilfenahme einer nach dem Gegenstromprinzip arbeitenden Extraktionsstufe (4) unter Durchmischung der Phasen verteilt, indem man das Ausgangspolyamingemisch über die wäßrige Phase in die Extraktionsstufe (4) einbringt und die die Extraktionsstufe (4) verlassende organische Phase (D)

b) zumindest teilweise über eine zwischengeschaltete Extraktionsstufe (3) und/oder

c) unter Abtrennung eines Teilstromes vor oder nach der durchlaufenden Extraktionsstufe (3) und Rückführung des abgetrennten Teilstromes zur Extraktionsstufe (4) über eine vorgeschaltete Extraktionsstufe (2)

d) in einer mehrstufigen Destillation (7.1), (7.2) in eine erste wiederverwendete Fraktion (E), bestehend im wesentlichen aus hydrophobem Lösungsmittel, eine zweite Fraktion (F), bestehend im wesentlichen aus Hilfsamin und einem Destillationsrückstand (G), bestehend im wesentlichen aus einer ersten Polyaminfraktion, auftrennt und

e) die die Extraktionsstufe (4) verlassende wäßrige Phase (H) in eine vorletzte Extraktionsstufe (5) leitet, in welcher nach dem Prinzip der Gegenstromextraktion eine Extraktion der wäßrigen Phase mit einer aus hydrophobem Lösungsmittel und Hilfsamin bestehenden Lösungsmittelphase (J) erfolgt, wobei die an Polyamin verarmte wäßrige Phase (K) resultiert, und

f) die in der Extraktionsstufe (5) anfallende organische Phase (L) zumindest teilweise in einer mehrstufigen Destillation (8.1), (8.2) in eine erste Fraktion (M), bestehend im wesentlichen aus hydrophobem Lösungsmittel und eine zweite Fraktion (N) bestehend im wesentlichen aus Hilfsamin und einen Destillationsrückstand (O), bestehend im wesentlichen aus einer zweiten Polyaminfraktion, auftrennt und

g) in der Extraktionsstufe (5) anfallende wäßrige Phase (K) zumindest teilweise,

h) zumindest teilweise über eine zwischengeschaltete Destillationsstufe (9)

i) in eine Extraktionsstufe (6) leitet und im Gegenstrom mit einer organischen Phase (P) extrahiert, bestehend im wesentlichen aus hydrophobem, daß in (6) eine gegenüber der eingesetzten wäßrigen Phase (K) an Arylamin verarmte wäßrige Phase (Q) resultiert, die man

k) zumindest teilweise über eine zwischengeschaltete Destillationsstufe (9)

l) mit den vorhandenen Resten von (Q) und (K) vereinigt und als Mengenstrom (C) wiederverwendet, indem man Mengenstrom (C)

m) zumindest teilweise über eine zwischengeschaltete Extraktionsstufe (3) und/oder

n) zumindest teilweise zunächst über eine vorgeschaltete Extraktionsstufe (2) und anschließend über eine gegebenenfalls vorhandene Extraktionsstufe (3)

o) zur Extraktionsstufe (4) zurückgeführt und dort als Mengenstrom (C) wiederverwendet und man

p) die in der Extraktionsstufe (6) anfallende organische Phase (R) als Extraktionsmittelphase (J) der Extraktionsstufe (5) zuführt.

**2.** Verfahren gemäß Anspruch 1, dadurch gekennzeichnet, daß der wiederverwendete Mengenstrom (C) einen höheren Protonierungsgrad aufweist als die die Extraktionsstufe (4) verlassende wäßrige Phase (K'),

**3.** Verfahren gemäß Anspruch 1 oder 2, dadurch gekennzeichnet, daß man

b) die in der Extraktionsstufe (4) anfallende organische Phase (D) zumindest teilweise in einer zwischengeschalteten Extraktionsstufe (3) mit zumindest einer Teilmenge des Mengenstrom (C) im Gegenstrom extrahiert und/oder
mit zumindest einer Teilmenge der in der vorgeschalteten Extraktionsstufe (2) anfallenden wäßrigen Phase (Z) im Gegenstrom extrahiert,
die in der zwischengeschalteten Extraktionsstufe (3) resultierende wäßrige Phase (U) der Extraktionsstufe (4) zuführt und
die in der zwischengeschalteten Extraktionsstufe (3) anfallende organische Phase (V) der Aufarbeitungsstufe (7) zuführt.

**4.** Verfahren gemäß einem oder mehreren der Ansprüche 1 bis 3, dadurch gekennzeichnet, daß man

c) einen Teilstrom der die Extraktionsstufe (4) verlassenden organischen Phase (D) und/oder
einen Teilstrom der die vorhandene zwischengeschaltete Extraktionsstufe (3) verlassenden organischen Phase abtrennt
und in einer vorgeschalteten Extraktionsstufe (2) mit einer Teilmenge, vorzugsweise mit der Gesamtmenge der als Mengenstrom (C) zur Verfügung stehenden wäßrigen Phase im Gegenstrom extrahiert,
den in der Extraktionsstufe (2) eingesetzten organischen Mengenstrom (W) so bemißt, daß in (2) ein möglichst weitgehender Übergang des im besagtem organischen Mengenstrom enthaltenen Polyamins in die wäßrige Phase erfolgt,
die in der vorgeschalteten Extraktionsstufe (2) resultierende wäßrige Phase (Z) zumindest teilweise der Extraktionsstufe (3) zuführt und
die in der vorgeschalteten Extraktionsstufe (2) anfallende an Polyamin verarmte organische Phase der Extraktionsstufe (4) zuführt.

**5.** Verfahren gemäß einem oder mehreren der Ansprüche 1 bis 4, dadurch gekennzeichnet, daß man

k) die die Extraktionsstufe (5) verlassende wäßrige Phase (K) vor ihrer Wiederverwendung zumindest teilweise und/oder die die Extraktionsstufe (6) verlassende wäßrige Phase (Q) vor ihrer Wiederverwendung zumindest teilweise destillativ (9) von einem Teil (X) des in ihr enthaltenen Wassers befreit, dieses zum Waschen (7.0) des der destillativen Aufarbeitung (7.1), (7.2) zugeführten Teiles der die Extraktionsstufe (4) verlassenden organischen Phase (D) und/oder der die Extraktionsstufe (3) verlassenden organischen Phase (V) und/oder

zum Waschen (8.0) des der destillativen Aufarbeitung (8.1), (8.2) zugeführten Teiles der die Extraktionsstufe (5) verlassenden organischen Phase (L) zwecks Entfernens von Säurespuren verwendet, das hierbei anfallende Wasser (Y) an geeigneter Stelle in die wäßrige Phase zurückführt, die resultierende konzentrierte wäßrige Phase mit den verbliebenen Resten von (K) und (Q) vereinigt und als Mengenstrom (C) der Wiederverwendung zuführt.

**6.** Verfahren gemäß einem oder mehreren der Ansprüche 1 bis 5, dadurch gekennzeichnet, daß man als Hilfsamin Anilin verwendet.

**7.** Verfahren gemäß einem oder mehreren der Ansprüche 1 bis 6, dadurch gekennzeichnet, daß man ein Polyamingemisch der Diphenylmethanreihe als Polyamingemisch verwendet, wie es bei der säurekatalysierten Anilin/Formaldehyd-Kondensation anfällt.

**8.** Verfahren zur Herstellung aromatischer Polyisocyanatgemische durch ein Verfahren enthaltend die folgenden Verfahrensstufen:

A) Herstellung aromatischer Polyamingemische der Diphenylmethanreihe gemäß den Ansprüchen 1-7,
B) Umwandlung der erhaltenen aromatischen Polyamingemische der Diphenylmethanreihe der Stufe (A) in die entsprechenden aromatischen Polyisocyanatgemische.

**9.** Verfahren zur Herstellung kernhydrierter Polyamine oder Vernetzer und Epoxidhärter durch ein Verfahren enthaltend die folgenden Verfahrensstufe:

A) Herstellung aromatischer Polyamingemische der Diphenylmethanreihe gemäß den Ansprüchen 1-7,
B) Umwandlung der erhaltenen aromatischen Polyamingemische der Diphenylmethanreihe der Stufe (A) in die entsprechenden kernhydriertes Polyamine oder Vernetzer und Epoxidhärter.

**10.** Verfahren zur Herstellung Polyurethankernstoffe durch ein Verfahren enthaltend die folgenden Verfahrensstufen :

A) Herstellung aromatischer Polyamingemische der Diphenylmethanreihe gemäß den Ansprüchen 1-7,
B) Umwandlung der erhaltenen aromatischen Polyamingemische der Diphenylmethanreihe der Stufe (A) in die entsprechenden Polyurethankunstoffe.

## Claims

**1.** Process for the fractionation and purification of aromatic polyamine mixtures of the diphenylmethane series, characterised in that

a) the polyamine starting mixture (A) is distributed, with intermixing of the phases, in a two-phase system comprising (i) a hydrophobic solvent phase (B) which substantially comprises hydrophobic solvent, and (ii) an aqueous phase (C) substantially comprising water, a strong acid and auxiliary amine which is present at least in part in the salt form, is virtually insoluble in water and exhibits at normal pressure a boiling point which is at least 20°C below the boiling point of the lowest-boiling component of the starting mixture and at least 20°C above the boiling point of the solvent, with the assistance of an extraction stage (4) operating on the countercurrent principle, in that the starting polyamine mixture is introduced into the extraction stage (4) by way of the aqueous phase, and the organic phase (D) leaving the extraction stage (4) is,

b) at least in part by way of an interposed extraction stage (3) and/or

c) with separation of a partial stream upstream or downstream of the extraction stage (3) which is passed through and return of the separated partial stream to the extraction stage (4) by way of an extraction stage (2) positioned upstream,

d) separated in a multi-stage distillation (7.1), (7.2) into a first, recycled fraction (E) substantially comprising hydrophobic solvent, a second fraction (F) substantially comprising auxiliary amine, and a distillation residue (G) substantially comprising a first polyamine fraction, and

e) the aqueous phase (H) leaving the extraction stage (4) is guided into a penultimate extraction stage (5) in which there takes place on the countercurrent extraction principle an extraction of the aqueous phase with a solvent phase (J) comprising hydrophobic solvent and auxiliary amine, wherein there results the aqueous phase (K) which is impoverished as to polyamine, and

f) the organic phase (L) arising in the extraction stage (5) is at least in part separated in a multi-stage distillation (8.1), (8.2) into a first fraction (M) substantially comprising hydrophobic solvent, and a second fraction (N) substantially comprising auxiliary amine, and a distillation residue (O) substantially comprising a second polyamine fraction, and

g) aqueous phase (K) arising in the extraction stage (5) is at least in part,

h) at least in part by way of an interposed distillation stage (9),

i) guided into an extraction stage (6) and is extracted in countercurrent with an organic phase (P) substantially comprising hydrophobic solvent, such that there results in (6) an aqueous phase (Q) which, compared with the aqueous phase (K) utilised, is impoverished as to arylamine and which is,

k) at least in part by way of an interposed distillation stage (9),

l) combined with the residues of (Q) and (K) which are present and is recycled as stream (C), in that stream (C) is,

m) at least in part by way of an interposed extraction stage (3) and/or

n) at least in part first by way of an extraction stage (2) positioned upstream and then by way of an optionally present extraction stage (3),

o) returned to the extraction stage (4) and is there recycled as stream (C), and

p) the organic phase (R) arising in the extraction stage (6) is supplied to the extraction stage (5) as the extraction agent phase (J).

**2.** Process according to Claim 1, characterised in that the recycled stream (C) exhibits a higher degree of protonation than the aqueous phase (K') leaving the extraction stage (4).

**3.** Process according to Claim 1 or 2, characterised in that

b) the organic phase (D) arising in the extraction stage (4) is at least in part extracted in countercurrent with at least a part volume of the stream (C) in an interposed extraction stage (3) and/or
is extracted in countercurrent with at least a part volume of the aqueous phase (Z) arising in the extraction stage (2) positioned upstream,
the aqueous phase (U) resulting in the interposed extraction stage (3) is supplied to the extraction stage (4), and the organic phase (V) arising in the interposed extraction stage (3) is supplied to the working-up stage (7).

**4.** Process according to one or more of Claims 1 to 3, characterised in that

c) a partial stream of the organic phase (D) leaving the extraction stage (4) and/or
a partial stream of the organic phase leaving the interposed extraction stage (3) which is present is separated and is extracted in countercurrent in an extraction stage (2) positioned upstream, with a part volume, preferably with the total volume, of the aqueous phase available as stream (C),
the organic stream (W) utilised in the extraction stage (2) is dimensioned such that there takes place in (2) as extensive a transfer as possible of the polyamine contained in the said organic stream into the aqueous phase, the aqueous phase (Z) resulting in the extraction stage (2) positioned upstream is supplied at least in part to the extraction stage (3), and the organic phase impoverished as to polyamine, which arises in the extraction stage (2) positioned upstream is supplied to the extraction stage (4).

**5.** Process according to one or more of Claims 1 to 4, characterised in that

k) before its recycling, the aqueous phase (K) leaving the extraction stage (5) is at least in part, and/or before its recycling the aqueous phase (Q) leaving the extraction stage (6) is at least in part, liberated by distillation (9) from a part (X) of the water contained therein, the latter is used for washing (7.0) that part of the organic phase (D) leaving the extraction stage (4) and/or of the organic phase (V) leaving the extraction stage (3), which is/are supplied to the working-up (7.1), (7.2) by distillation, and/or for washing (8.0) that part of the organic phase (L) leaving the extraction stage (5), which is supplied to the working-up (8.1), (8.2) by distillation, for the purpose of removing traces of acid, the water (Y) arising thereby is returned at a suitable point into the aqueous phase, and the resulting concentrated aqueous phase is combined with the remaining residues of (K) and (Q) and is supplied to recycling as stream (C).

**6.** Process according to one or more of Claims 1 to 5, characterised in that aniline is used as the auxiliary amine.

**7.** Process according to one or more of Claims 1 to 6, characterised in that a polyamine mixture of the diphenylmethane series such as occurs during acid-catalysed aniline formaldehyde condensation is used as the polyamine mixture.

**8.** Process for the preparation of aromatic polyisocyanate mixtures by a process comprising the following process steps:

A) preparation of aromatic polyamine mixtures of the diphenylmethane series according to Claims 1 - 7,
B) conversion of the aromatic polyamine mixtures of the diphenylmethane series which have been obtained in the stage (A) into the corresponding aromatic polyisocyanate mixtures.

**9.** Process for the preparation of ring-hydrogenated polyamines or cross-linking agents and epoxy hardeners by a process comprising the following process steps:

A) preparation of aromatic polyamine mixtures of the diphenylmethane series according to Claims 1 - 7,
B) conversion of the aromatic polyamine mixtures of the diphenylmethane series which have been obtained in the stage (A) into the corresponding ring-hydrogenated polyamines or cross-linking agents and epoxy hardeners.

**10.** Process for the preparation of polyurethane plastics by a process comprising the following process steps:

A) preparation of aromatic polyamine mixtures of the diphenylmethane series according to Claims 1 - 7,
B) conversion of the aromatic polyamine mixtures of the diphenylmethane series which have been obtained in the stage (A) into the corresponding polyurethane plastics.

**Revendications**

**1.** Procédé pour le fractionnement et la purification de mélanges de polyamines aromatiques de la série des diphénylméthanes, caractérisé en ce que

a) on répartit le mélange de polyamines de départ (A) en un système biphasique constitué par (i) une phase de solvant hydrophobe (B) qui est constituée essentiellement d'un solvant hydrophobe et par (ii) une phase aqueuse (C) constituée essentiellement par de l'eau, par un acide fort et par une amine auxiliaire présente au moins en partie sous forme saline, qui est pratiquement insoluble dans l'eau et qui présente, sous pression normale, un point d'ébullition d'au moins 20°C inférieur au point d'ébullition du composant du mélange de départ possédant le point d'ébullition le plus bas et d'au moins 20°C supérieur au point d'ébullition du solvant, à l'aide d'une étape d'extraction (4) travaillant conformément au principe d'un écoulement à contre-courant, avec mélange intime des phases, en introduisant le mélange de polyamines de départ, via la phase aqueuse, dans l'étape d'extraction (4) et on sépare la phase organique (D) quittant l'étape d'extraction (4),

b) au moins en partie via une étape d'extraction (3) intercalée et/ou

c) en séparant un courant partiel avant ou après son passage par l'étape d'extraction (3) et en renvoyant le courant partiel séparé à l'étape d'extraction (4) via une étape d'extraction (2) montée en amont,

d) dans une distillation en plusieurs étapes (7.1), (7.2), en une première fraction recyclée (E) constituée essentiellement par le solvant hydrophobe, en une deuxième fraction (F) constituée essentiellement par l'amine auxiliaire et en un résidu de distillation (G) constitué essentiellement par une première fraction de polyamine, et

e) on guide la phase aqueuse (H) quittant l'étape d'extraction (4), dans une avant-dernière étape d'extraction (5), dans laquelle a lieu, conformément au principe de l'extraction à contre-courant, une extraction de la phase aqueuse dans une phase de solvant (J) constituée par un solvant hydrophobe et par une amine auxiliaire, dans laquelle on obtient la phase aqueuse (K) appauvrie en polyamines, et

f) on sépare la phase organique (L) obtenue dans l'étape d'extraction (5), au moins en partie dans une distillation en plusieurs étapes (8.1), (8.2), en une première fraction (M) constituée essentiellement d'un solvant hydrophobe et en une deuxième fraction (N) constituée essentiellement de l'amine auxiliaire, et en un résidu de distillation (O) constitué essentiellement d'une deuxième fraction de polyamine, et

g) on guide la phase aqueuse (K) obtenue dans l'étape d'extraction (5), au moins en partie,

h) au moins en partie [sic] via une étape de distillation (9) intercalée,

i) dans une étape d'extraction (6) et on l'extrait à contre-courant avec une phase organique (P) constituée essentiellement d'un hydrophobe [sic], si bien que l'on obtient dans (6) une phase aqueuse (Q) appauvrie en arylamine par rapport à la phase aqueuse (K) mise en oeuvre, que l'on combine

k) le cas échéant, au moins en partie via une étape de distillation (9) intercalée,

l) avec les résidus éventuellement présents de (Q) et de (K), et que l'on recycle sous forme de courant massique (C), en renvoyant le courant massique (C)

m) au moins en partie via une étape d'extraction (3) intercalée et/ou

n) au moins en partie, d'abord via une étape d'extraction (2) montée en amont et ensuite, via une étape d'extraction (3) éventuellement présente,

o) à l'étape d'extraction (4), et on l'y recycle sous forme de courant massique (C), et

p) on achemine à l'étape d'extraction (5) la phase organique (R) obtenue dans l'étape d'extraction (6), sous forme d'une phase (J) contenant un agent d'extraction.

**2.** Procédé selon la revendication 1, caractérisé en ce que le courant massique recyclé (C) présente un degré de protonation supérieur à celui de la phase aqueuse (K') quittant l'étape d'extraction (4).

**3.** Procédé selon la revendication 1 ou 2, caractérisé en ce que

b) on extrait à contre-courant la phase organique (D) obtenue à l'étape d'extraction (4) au moins en partie dans une étape d'extraction (3) intercalée, avec au moins une quantité partielle du courant massique (C), et/ou on l'extrait à contre-courant avec au moins une quantité partielle de la phase aqueuse (Z) obtenue dans l'étape d'extraction (2) montée en amont,
on achemine à l'étape d'extraction (4) la phase aqueuse (U) résultant de l'étape d'extraction (3) intercalée, et on achemine à l'étape de traitement (7) la phase organique (V) obtenue dans l'étape d'extraction (3) intercalée.

**4.** Procédé selon une ou plusieurs des revendications 1 à 3, caractérisé en ce que

c) on sépare un courant partiel de la phase organique (D) quittant l'étape d'extraction (4) et/ou
un courant partiel de la phase organique quittant l'étape d'extraction (3) intercalée présente,
et on l'extrait à contre-courant dans une étape d'extraction (2) montée en amont, avec une quantité partielle, de préférence avec la quantité totale de la phase aqueuse disponible à titre de courant massique (C),
on proportionne le courant massique organique (W) mis en oeuvre à l'étape d'extraction (2) de telle sorte que l'on obtient dans (2) un transfert le plus étendu possible de la polyamine contenue dans le courant massique organique mentionné en phase aqueuse,

on achemine la phase aqueuse (Z) résultant de l'étape d'extraction (2) montée en amont, au moins en partie, à l'étape d'extraction (3), et
on achemine à l'étape d'extraction (4) la phase organique appauvrie en polyamines obtenue à l'étape d'extraction (2) montée en amont.

**5.** Procédé selon une ou plusieurs des revendications 1 à 4, caractérisé en ce que

k) on libère au moins en partie, par distillation (9), la phase aqueuse (K) quittant l'étape d'extraction (5), avant son recyclage, au moins en partie, et/ou la phase aqueuse (Q) quittant l'étape d'extraction (6), avant son recyclage, d'une partie (X) de l'eau qu'elle contient et que l'on utilise pour le lavage (7.0) de la partie acheminée au traitement par distillation (7.1), (7.2) de la phase organique (D) quittant l'étape d'extraction (4) et/ou de la phase organique (V) quittant l'étape d'extraction (3) et/ou pour le lavage (8.0) de la partie acheminée au traitement par distillation (8.1), (8.2) de la phase organique (L) quittant l'étape d'extraction (5), à des fins d'élimination des traces d'acide, on recycle l'eau (Y) que l'on obtient en l'occurrence, à l'endroit approprié dans la phase aqueuse, on combine la phase aqueuse concentrée résultante, avec les résidus subsistants de (K) et de (Q), et on les achemine au recyclage sous forme du courant massique (C).

**6.** Procédé selon une ou plusieurs des revendications 1 à 5, caractérisé en ce qu'on utilise, à titre d'amine auxiliaire, l'aniline.

**7.** Procédé selon une ou plusieurs des revendications 1 à 6, caractérisé en ce qu'on utilise, à titre de mélange de polyamines, un mélange de polyamines de la série des diphénylméthanes, tel qu'on l'obtient lors de la condensation d'aniline/formaldéhyde catalysée par un acide.

**8.** Procédé pour la préparation de mélanges de polyisocyanates aromatiques en passant par un procédé comprenant les étapes opératoires ci-après:

A) préparation de mélanges de polyamines aromatiques de la série des diphénylméthanes selon les revendications 1 à 7,
B) transformation des mélanges de polyamines aromatiques obtenus de la série des diphénylméthanes de l'étape (A) en mélanges de polyisocyanates aromatiques correspondants.

**9.** Procédé pour la préparation de polyamines hydrogénées au noyau ou encore d'agents de réticulation et de durcisseurs époxy via un procédé comprenant les étapes opératoires ci-après:

A) préparation de mélanges de polyamines aromatiques de la série des diphénylméthanes selon les revendications 1 à 7,
B) transformation des mélanges de polyamines aromatiques obtenus de la série des diphénylméthanes de l'étape (A) en polyamines correspondantes hydrogénées au noyau ou bien en agents de réticulation et en durcisseurs époxy.

**10.** Procédé pour la préparation de matières synthétiques de polyuréthanne via un procédé comprenant les étapes opératoires ci-après:

A) préparation de mélanges de polyamines aromatiques de la série des diphénylméthanes selon les revendications 1 à 7,
B) transformation des mélanges de polyamines aromatiques obtenus de la série des diphénylméthanes de l'étape (A) en matières synthétiques de polyuréthanne correspondantes.

Fig.1

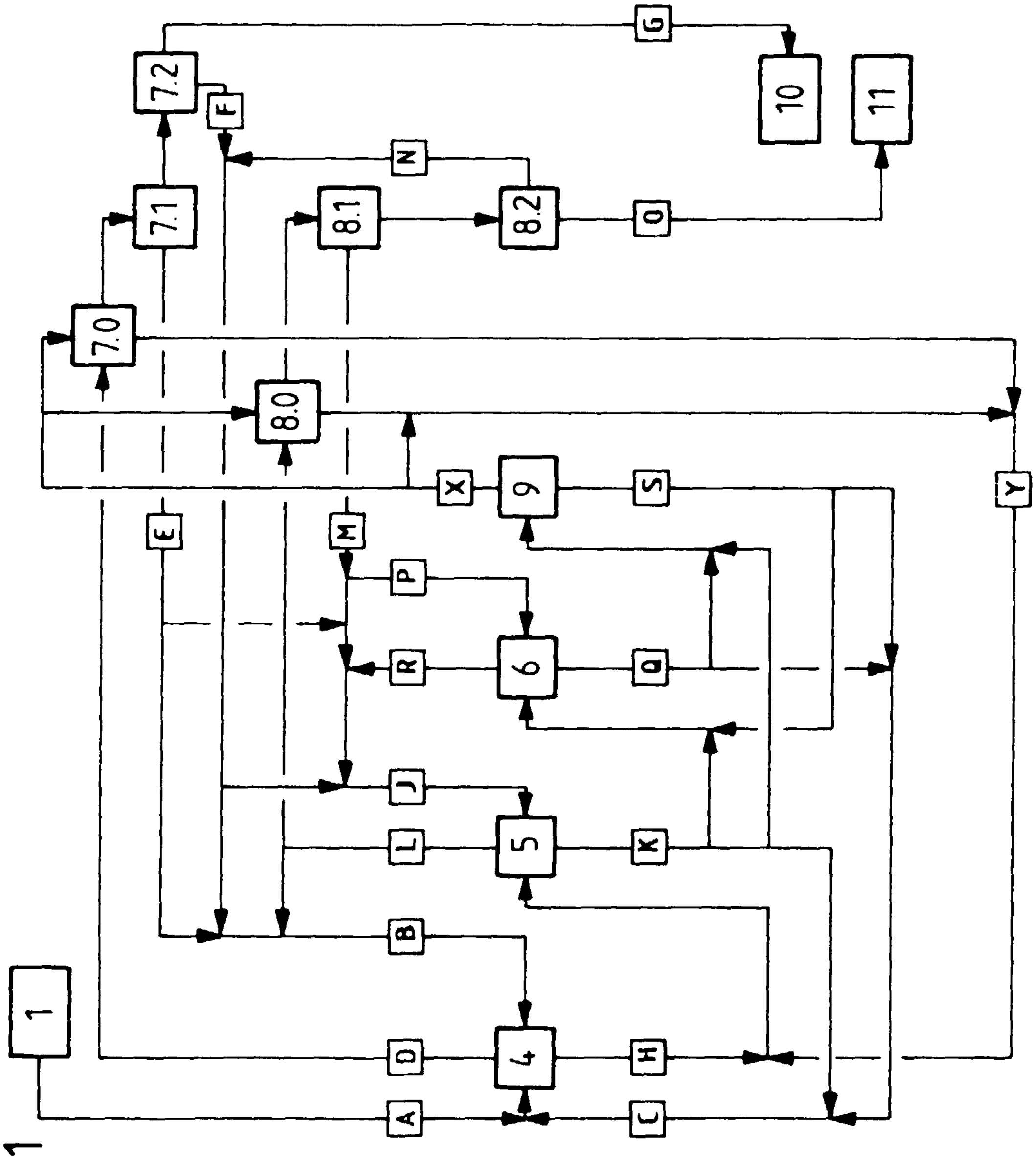

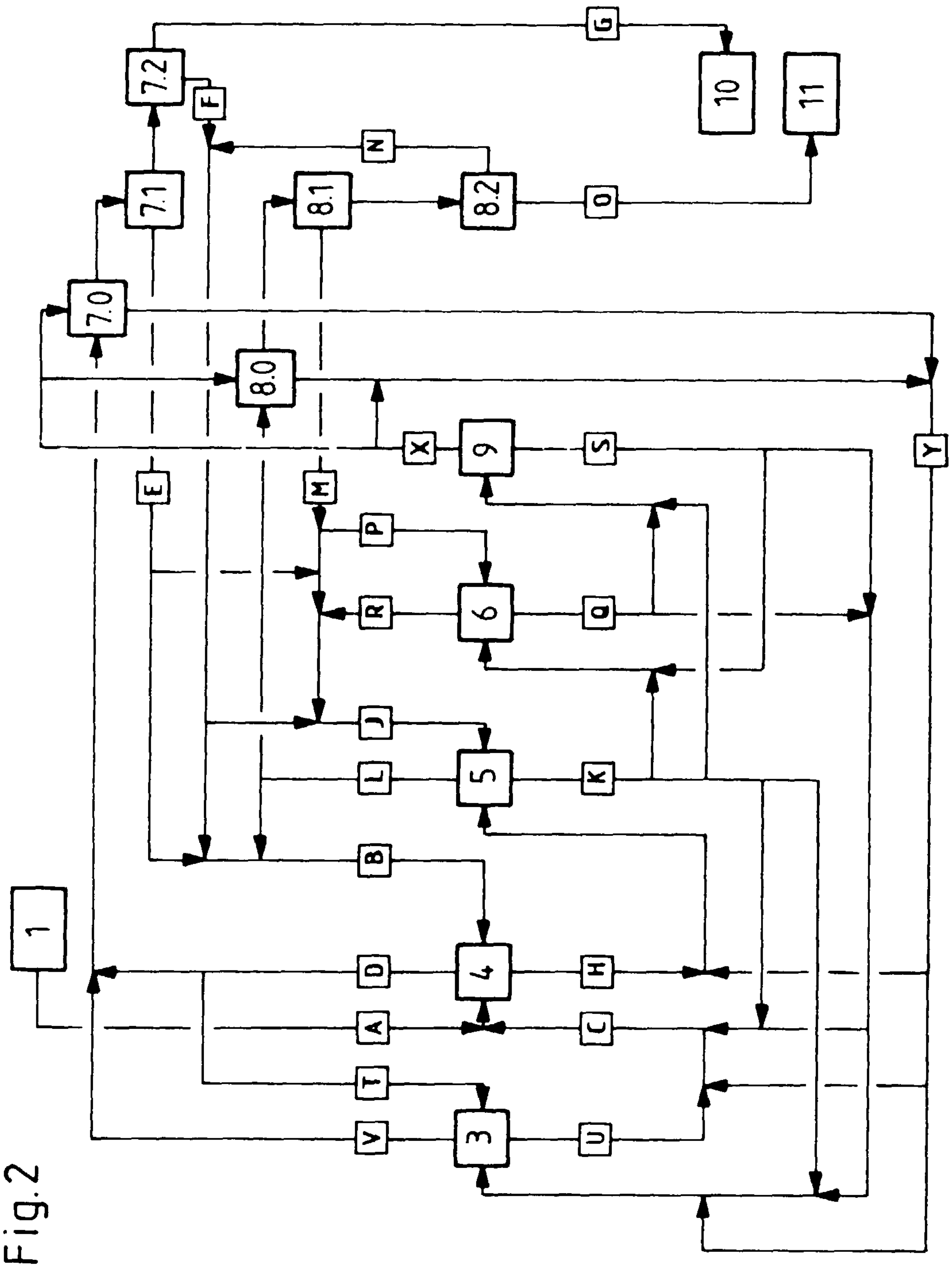


Fig.2

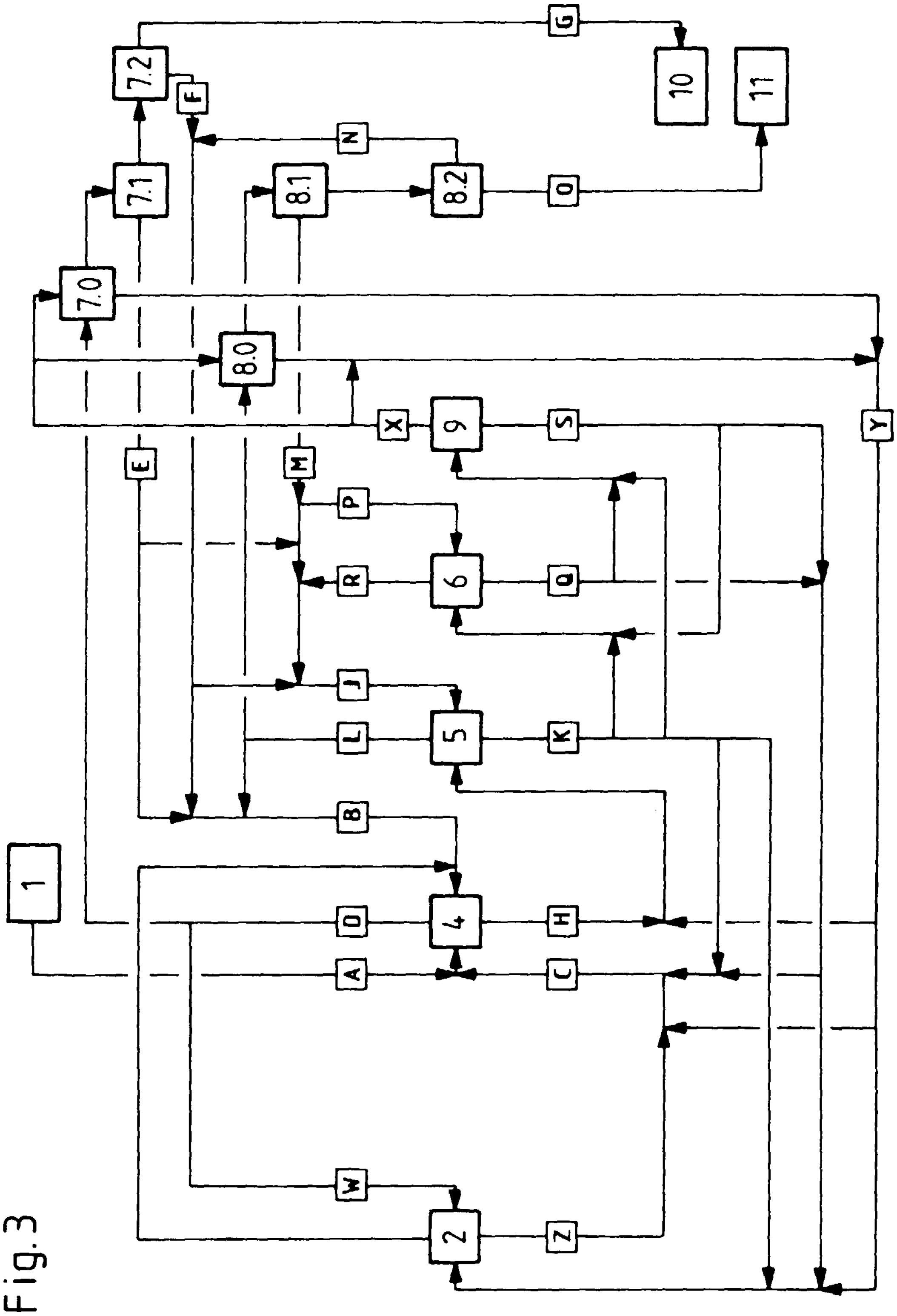


Fig. 3

Fig. 4